# EUROPEAN PATENT APPLICATION

(11) **EP 2 386 562 A1**
(43) Date of publication of application: **16.11.2011**
(21) Application number: 09837468.9
(22) Date of filing: 25.12.2009
(51) Int. Cl.: C07H 1/00, A61K 51/00, B01J 3/00, B01J 19/00, B81B 1/00, C07H 5/02

(54) **HALOGEN-SUBSTITUTED SACCHARIDE, METHOD FOR PRODUCING SAME, REACTION COMPOSITION OF SAME AND DEVICE FOR PRODUCING SAME**

(30) Priority: 07.01.2009 JP 2009002079; 10.12.2009 JP 2009281038
(71) Applicant: National Institute of Advanced Industrial Science and Technology, Tokyo 100-8921 (JP)
(72) Inventor: SATO, Masahiro, Sendai-shi Miyagi 983-8551 (JP); KAWANAMI, Hajime, Sendai-shi Miyagi 983-8551 (JP); MIZUKAMI, Fujio, Sendai-shi Miyagi 983-8551 (JP)
(74) Representative: Jansen, Cornelis Marinus
(86) International application number: PCT/JP2009/007314
(87) International publication number: WO 2010/079579

(57) **Abstract**

The present invention provides: a method for synthesizing a halogen-substituted saccharide from a leaving group-substituted saccharide in a short-time and continuously; a reaction composition; and a device for synthesis of same, i.e., the method being for synthesizing a halogen-substituted saccharide from a leaving group-substituted saccharide by a halogen-substituted saccharide synthesis reaction using a subcritical fluid or a supercritical fluid as the reaction solvent; the reaction composition of the same being an aqueous solution of halogen-substituted saccharide; and the device for synthesis being a device for producing the same, the method being for producing a halogen-substituted saccharide with a subcritical fluid or a supercritical fluid at a temperature of 100 to 400°C and a pressure of 0.1 to 40 MPa or a mixed solvent of aprotic organic solvent or inorganic solvent mixed therein as the reaction solvent, under a catalyst-free condition, by introducing the leaving group-substituted saccharide and the reaction solvent into a circulating-type high-temperature and high-pressure device to produce selectively a halogen-substituted saccharide at high yield, high selectivity, high speed and continuously, while reducing the amount of energy consumption and the amount of waste.

## Description

### TECHNICAL FIELD

The present invention relates to a halogen-substituted saccharide, a method for producing same, a reaction composition and a device for producing same, and more specifically, to a technology for producing a halogen-substituted saccharide with water or ethanol or a mixed solvent thereof at a high temperature and high pressure state as a reaction solvent, with no catalyst and in a single step. The present invention provides a method for continuously synthesizing a halogen-substituted saccharide from a leaving group-substituted protected saccharide or a leaving group-substituted saccharide and a halide salt, in a single step and in a short time, with water at ordinary temperature or water at a temperature of 100 to 400°C and a pressure of 0.1 to 40 MPa, or a water-mixed solvent as the reaction solvent, with no addition of a catalyst; a reaction composition of same; and a device for producing same. Here, as halogen atoms in the halogen-substituted saccharide, fluorine, chlorine, bromine and iodine may be cited.

### BACKGROUND ART

Halogen-substituted saccharides are useful in the pharmaceutical field, as the products have increased functionality and added value compared to the substrates/raw materials. For instance, [¹⁸F]-2-fluoro-2-deoxyglucose ([¹⁸F]-FDG), which contains radioactive fluorine, is used as a radiochemical tracer (110 minutes half life) in positron emission tomography (PET), allowing abnormal sites to be identified/evaluated readily in the fields of oncology, neurology and cardiology.

Concretely, PET is used for the measurement of glucose metabolism in tissues such as brain and cardiac muscle, provides images for real time diagnosis and management, and in addition, by concentrating in cancer cells, which have high metabolism, enables identification of small cancer cells, and can be used in tumor disease research, such as early stage discovery of cancer. Furthermore, novel applications are being found for PET in the field of drug discovery development.

Conventionally, when synthesizing a halogen-substituted saccharide from a leaving group-substituted protected saccharide or a leaving group-substituted saccharide and a halide salt by a nucleophilic substitution reaction, since halogen salts do not dissolve in aprotic organic solvent, a phase transfer catalyst is required; since removal of the remaining aprotic organic solvent and catalyst, which are harmful to the organism, require large effort and energy, not only are there problems such as of affecting the environment, but also of being harmful to the organism.

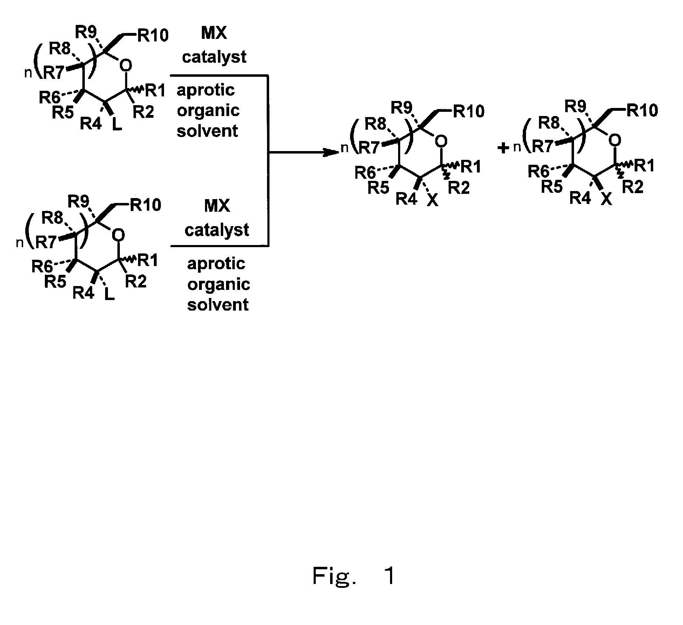

In prior art, a variety of methods for synthesizing a halogen-substituted saccharide from a leaving group-substituted protected saccharide by a nucleophilic substitution reaction using an aprotic organic solvent and a catalyst have been reported as prior art techniques (for instance, refer to Non-patent Reference 1). Here, protection is the substitution of a hydroxyl group by a protecting group, the elimination of which is difficult compared to a leaving group, and as protecting groups, acyl protecting groups such as acetoxy group and benzoyloxy group, alkyl group, benzyl group, ether protecting groups such as methoxy methyl group and acetal group, silyl protecting group such as tetramethyl silyl group, and the like, may be cited. If a nucleophilic substitution reaction technique for synthesizing a halogen-substituted monosaccharide from a leaving group-substituted protected monosaccharide is completed, halogen substitution would become possible also in disaccharides, trisaccharides and polysaccharides, such that a technique for synthesizing a halogen substituted monosaccharide has been reported (Fig. 1).

In Fig. 1, when halogen substitution is to position 2 of the pyranoside with n = 1, R1, R2, R3, R4, R5, R6, R7, R8, R9 and R10 represent a hydrogen or a hydroxyl group or a protecting group such as acetoxy group or another saccharide substituent, L represents a leaving group, M represents a metal atom and X represents a halogen atom. Similarly, in Fig. 1, when halogen substitution is to position 2 of the fructoside with n = 0, R1, R2, R3, R4, R5, R6, R9 and R10 represent a hydrogen or a hydroxyl group or a protecting group such as an acetoxy group or another saccharide substituent, L represents a leaving group, M represents a metal atom and X represents a halogen atom.

According to prior art references, a method has been proposed in which fluorine ion is transferred from an aqueous solution of potassium fluoride into the aprotic organic solvent acetonitrile using a phase transfer catalyst such as cryptand to substitute position 2 of tetraacetyl-(D)-mannose-2-triflate with fluorine and obtain tetraacetyl-2-fluoro-2-deoxy-(D)-glucose, and then by deprotection, 2-fluoro-2-deoxy-(D)-glucose is synthesized with a yield of 80% (Non-patent Reference 2).

In addition, a method in which this synthesis method is performed in a micro-reactor to obtain 2-F-FDG with a yield of 90% (Non-patent Reference 3), or a method in which, with a support comprising a mannose derivative supported with polyperfluorosulfonate at position 2 of the mannose derivative protected by protecting groups at positions 1, 3, 4 and 5 thereof as raw materials, and cryptand as the phase transfer catalyst, position 2 is fluorine-substituted in acetonitrile, and then deprotection is performed to synthesize 2-fluoro-2-deoxy-(D)-glucose with a yield of 80 to 90% (Non-patent Reference 4), and the like, have been proposed.

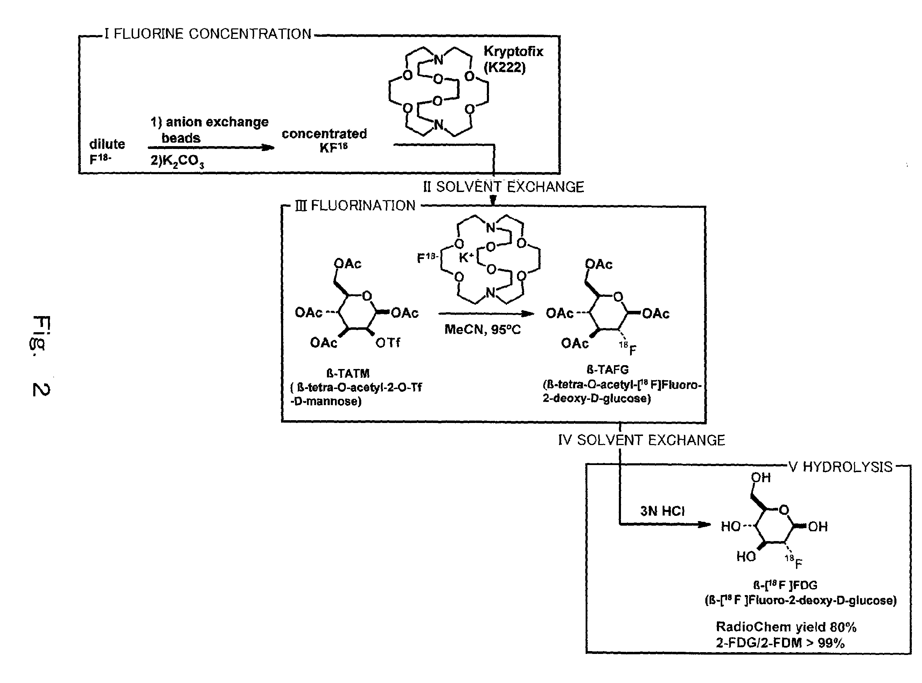

In the above prior art technique, the use of a phase transfer catalyst such as cryptand, for instance, kryptofix K222, and performance of solvent substitution and use of aprotic organic solvent that accompanies this are indispensable, and if purification for obtaining a high purity target compound is also included, an operation spanning multiple steps becomes necessary. A synthesis route for a halogen-substituted saccharide from a leaving group-substituted saccharide and a halide salt is shown in Fig. 2.

In addition, in the post-processing following the reaction, in the case of a prior art technique, after neutralization by adding a neutralization agent to the reaction mixture, an extraction solvent and water or saturated sodium chloride water are added to perform liquid separation, the solvent layer thereafter undergoes processes of drying, solvent elimination, distillation or rectification to obtain the target compound; however, in the aqueous layer, in addition to water, a complex mixture of catalyst, aprotic organic solvent, substrate raw materials, products, byproducts and inorganic compounds is contained.

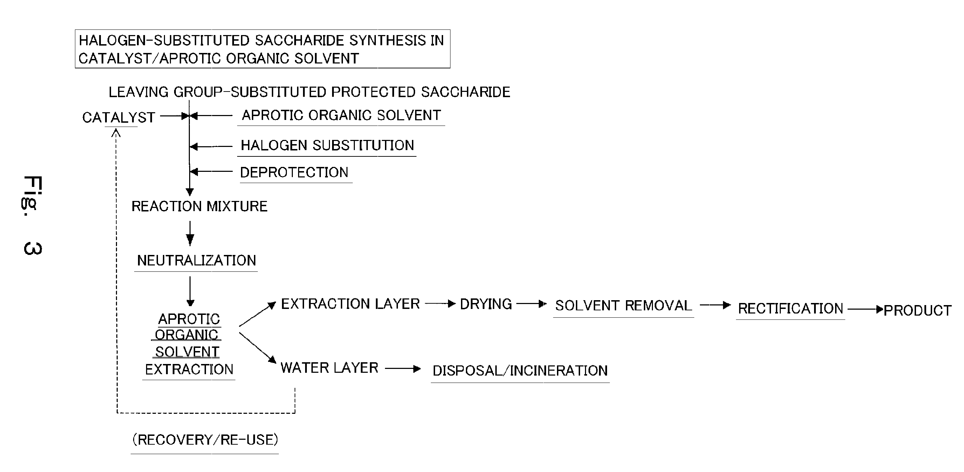

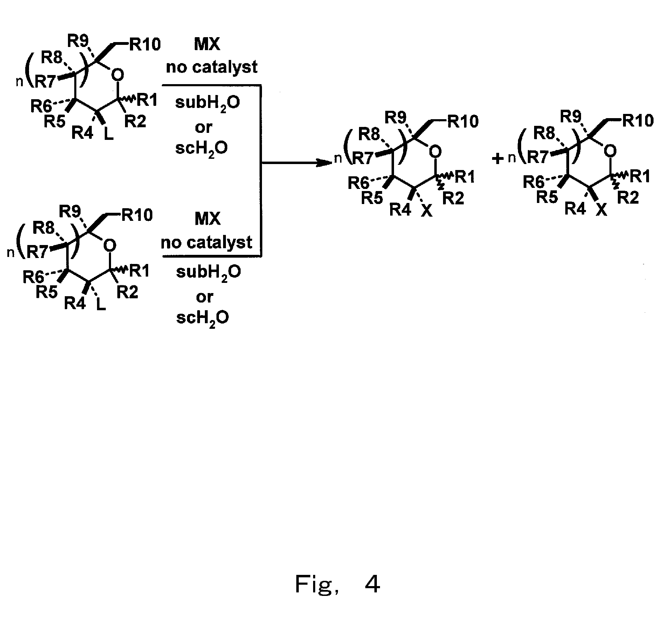

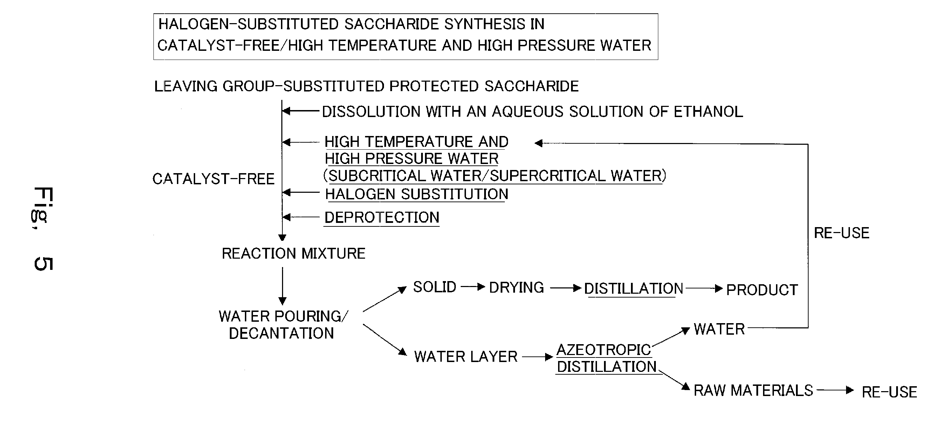

Here, if separation of the catalyst from the aqueous layer is straightforward, the catalyst is recovered, regenerated and re-used; if separation thereof is difficult, it is discarded/disposed of as-is. A synthesis flow chart for a halogen-substituted saccharide using catalyst/aprotic organic solvent is shown in Fig. 3. In addition, a post-processing flow chart for a halogen-substituted saccharide using catalyst/aprotic organic solvent is shown in Fig. 4. As with a halogen-substituted saccharide synthesis in catalyst-free/high temperature and high pressure water (Fig. 4), if the aqueous layer contains water and products only, and does not contain catalyst and aprotic organic solvent, the products can be separated by decantation alone. This means that the process enables regeneration of water and is of an environmentally friendly type compared to prior art techniques. A post-processing flow chart for a halogen-substituted saccharide using catalyst-free/water solvent is shown in Fig. 5.

### Prior Art References

### Non Patent References

Non-patent Reference 1: H.H. Coenen, V.W. Pike, G. Stocklin and R. Wagner, Appl. Radiat. Isotr; 1987, 38(8), 605
Non-patent Reference 2: K. Hamacher, H.H. Coenen, and G. Stocklin, J. Nucl. Med., 1986, 27, 235
Non-patent Reference 3: C.-C. Lee, G. Sui, A. Elizarov, C.J. Shu, Y.-S. Shin, A.N. Dooley, J. Huang, A. Daridon, P. Wyatt, D. Stout, H.C. Kolb, O.N. Witte, N. Satyamurthy, J.R. Heath, M.E. Phelps, S.R. Quake and H.-R. Tseng, Science, 2008, 310, 1793
Non-patent Reference 4: L.J. Brown, D.R. Bouvet, S. Champion, A.M. Gibson, Y. Hu, A. Jackson, I. Khan, N. Ma, N. Millot, H. Wadsworth, and R.C. Brown, Angew. Chem. Int. Ed., 2007, 46, 941

In this way, with prior art methods, in the case of halogen-substituted saccharide synthesis, a catalyst such as a phase transfer catalyst and an aprotic organic solvent are required, which, from the standpoint of product quality, necessitates the removal of the catalyst and the aprotic organic solvent in a separation operation following the reaction, and the aqueous layer after the separation operation is prone to become a waste, giving rise to the problem of liquid wastes. Furthermore, from the considerations of the effects on the environment and hazardousness towards ecosystems, and in addition, from the point of view of safety as a medicinal product ingested by a human, a higher degree of separation of the catalyst/aprotic organic solvent is necessary. As the costs required for a high degree separation is on the same order as for the synthesis operation, it is desirable that no catalyst and no aprotic organic solvent be used.

From the above, in the technical field, development of a synthesis method is in strong demand in which, with a simple, low cost, environmentally friendly synthesis process, separation operation is straightforward, allowing for a high degree of separation, and enabling continuous synthesis of a halogen-substituted saccharide in which no catalyst or aprotic organic solvent remains.

### Description of Invention

### Subject to be Resolved by Invention

In this situation, the present inventors, as a result of earnest studies, in view of the above prior art techniques, with the purpose of developing a novel synthesis method capable of synthesizing the above halogen-substituted saccharide continuously and selectively by a low cost, environment-friendly, simple high-performance synthesis process, discovered that a halogen-substituted saccharide could be synthesized selectively from a leaving group-substituted saccharide and a halide salt by a nucleophilic substitution reaction without a catalyst when a high-temperature and high-pressure water, or a subcritical water or a supercritical water is the reaction solvent, and reached completion of the present invention.

### DISCLOSURE OF THE INVENTION

It is an object of the present invention to provide a method for synthesizing a halogen-substituted saccharide from a leaving group-substituted saccharide or a leaving group-substituted protected saccharide and a halide salt, continuously, under catalyst-free and short-time reaction conditions, and a reaction composition of same. It is an object of the present invention to provide a method for synthesizing a halogen-substituted saccharide from a leaving group-substituted protected saccharide or a leaving group-substituted saccharide and a halide salt only with a process using water with no catalyst, and a reaction composition of same, and to provide a method for producing a halogen-substituted saccharide which is applicable not only to pharmaceutical products but also to chemical product synthesis, and the like, allowing a halogen-substituted saccharide to be produced and provided with satisfactory yields in a short time and in large amounts, without affecting the environment and ecosystem, and a device for producing same.

### Means for Solving Subject

The present invention for solving the problems described above is an aqueous solution of halogen-substituted saccharide which is synthesized by a halogen-substituted saccharide synthesis reaction from a leaving group-substituted protected saccharide or a leaving group-substituted saccharide and a halide salt, and which contains a halogen substitution reaction product or contains a halogen-substituted saccharide for a tracer in positron emission tomography (PET), and in which there are no remaining catalyst and aprotic organic solvent harmful to humans and organisms.

In addition, the present invention is a method for producing a halogen-substituted saccharide, which uses high-temperature and high-pressure water, or a subcritical fluid or a supercritical fluid in a high-temperature and high-pressure state as the reaction solvent to carry out, from a leaving group-substituted protected saccharide and a halide salt, halogen substitution and deprotection in a single step, without substituting the solvent and with no catalyst, thereby synthesizing a halogen-substituted saccharide selectively.

Further, the present invention is a method for producing a halogen-substituted saccharide, which uses high-temperature and high-pressure water, or a subcritical fluid or a supercritical fluid in a high-temperature and high-pressure state as the reaction solvent to carry out, from a leaving group-substituted saccharide and a halide salt, halogen substitution in a single step without substituting the solvent and with no catalyst, thereby synthesizing a halogen-substituted saccharide selectively.

The method of the present invention has the following preferred aspects: 1) to use high-temperature and high-pressure water, or a subcritical fluid or a supercritical fluid at a temperature of 100 to 400°C and a pressure of 0.1 to 40 MPa as the reaction solvent, 2) to use water, ethanol, or another inorganic solvent, or an organic solvent or a mixed solvent of an inorganic solvent and an organic solvent, as subcritical fluid or supercritical fluid, 3) to perform the synthesis reaction by introducing the substrate and the reaction solvent into a circulating-type high temperature and high pressure device and varying the reaction time in the range of 3 to 180 seconds, and 4) to add an additive that is harmless to humans and organisms such as sodium hydrogen carbonate (sodium bicarbonate) in a method for synthesizing a halogen-substituted saccharide to accelerate the synthesis reaction.

In addition, the present invention is a halogen-substituted saccharide synthesizer, which is a micro reaction system in which the flow-path space is a micro space, the synthesizer being provided with a water solution-sending pump for sending water, a water heating coil, a high-temperature and high-pressure flow-cell, a reactant solution-sending pump for sending a substrate, a furnace body, a reactant inlet tube for introducing a reactant into the furnace body, a discharge solution line for discharging the reaction solution, a cooling flange and a back-pressure valve for setting the pressure, wherein the flow of an aqueous solution in which a leaving group-substituted protected saccharide and a halogen salt have been dissolved or the flow of an aqueous solution in which a leaving group-substituted saccharide and a halogen salt have been dissolved is collided at a right angle against the flow of high temperature and high pressure water in the high temperature and high pressure flow-cell, thereby synthesizing halogen-substituted saccharide selectively, and furthermore, is a method for a simple continuous solid-liquid separation of a solid containing the halogen-substituted saccharide by which, after halogen-substituted saccharide synthesis, water is poured onto the recovered aqueous solution for decantation, and after separation into solid-liquid bilayer solutions, the solid containing the halogen-substituted saccharide is separated and recovered in one operation.

Hereinafter, the present invention will be described in detail.
The present invention synthesizes a halogen-substituted saccharide of Chem. 4 or Chem. 5 from a leaving group-substituted saccharide of Chem. 1 or Chem. 2 selectively and continuously by a single step reaction process under the reaction conditions of no catalyst addition and in a short time. In the present invention high temperature and high pressure water, or a subcritical fluid or a supercritical fluid at a temperature of 100 to 400°C and a pressure of 0.1 to 40 MPa is used as the reaction solvent, and preferably, subcritical water is used. In addition, reaction conditions are adjusted preferably to a temperature of 200°C, a pressure of 5 MPa, reaction time in the range of 3 to 180 seconds, and preferably a reaction time of on the order of 10 seconds.

Here, in formulae Chem. 1, Chem. 2, Chem. 4 and Chem. 5, n = 0 represents a fructoside, R1, R2, R3, R4, R5, R6, R9 and R10 represent a hydrogen or a hydroxyl group or a protecting group such as an acetoxy group or a sugar substituent, L represents a leaving group, n = 1 represents a pyranoside, R1, R2, R3, R4, R5, R6, R7, R8, R9 and R10 represent a hydrogen or a hydroxyl group or a protecting group such as an acetoxy group or a sugar substituent, L represents a leaving group, in formula Chem. 3, M represents a metal cation such as lithium, sodium and potassium or an inorganic cation such as ammonium ion, and X represents a halogen ion such as fluorine or chlorine or bromine or iodine.

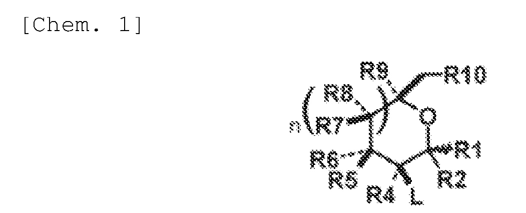

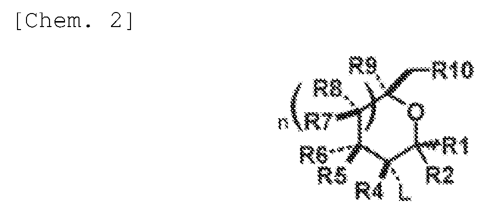

[Chem. 3] **MX**

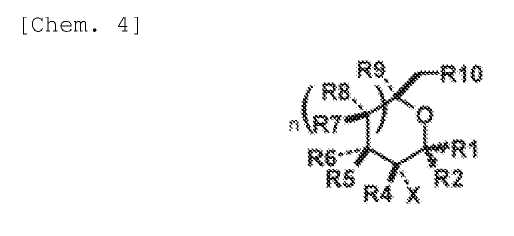

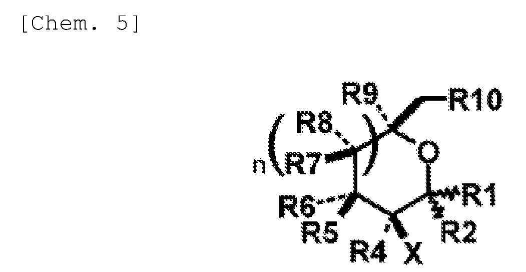

In the present invention, the above-mentioned substrate and reaction solvent are introduced into the reaction container to perform a prescribed synthesis reaction in a prescribed reaction time. Consequently, as the reactor, for instance, batch-type ordinary-temperature, high-pressure device or high temperature and high pressure reaction container, and continuous circulating-type ordinary-temperature, high-pressure device or circulating-type high temperature and high pressure reaction device can be used; however, in the present invention, there is no particular limitation to these reaction device formats.

In the method of the present invention the above ordinary temperature liquid or subcritical fluid or supercritical fluid existing in a high temperature and high pressure state is used as the reaction solvent; concretely, subcritical carbon dioxide (ordinary temperature or higher and 0.1 MPa or higher), subcritical water (100°C or higher and 0.1 MPa or higher), subcritical methanol (100°C or higher and 0.1 MPa or higher), subcritical ethanol (100°C or higher and 0.1 MPa or higher), supercritical carbon dioxide (34°C or higher and 7.38 MPa or higher), supercritical water (375°C or higher and 22 MPa or higher), supercritical methanol (239°C or higher and 8.1 MPa or higher), supercritical ethanol (241°C or higher and 6.1 MPa or higher), same state mixed solvents can be given as examples, and preferably, subcritical water (200 to 250°C and 5 MPa or higher) is used.

As the reaction solvent, an organic solvent or an inorganic solvent other than those mentioned above can be contained in an arbitrary proportion, and concretely, substituting with a reaction solution containing ethanol, methanol, acetone, acetonitrile, tetrahydrofuran, or the like, as organic solvent, or acetic acid, ammonia, or the like, as inorganic solvent, is possible.

In the present invention, the reaction products can be synthesized efficiently in a short time by adjusting the composition of the reaction solvent of the ordinary temperature liquid, subcritical fluid and supercritical fluid, the temperature and pressure conditions, the type of substrate and the amount thereof to be used, and the reaction time. In addition, in the present invention the prescribed reaction products can be synthesized, for instance, by introducing the substrate and reaction solvent into a circulating-type high temperature and high pressure device and changing the reaction time thereof in the range of 3 to 180 seconds. The above reaction conditions can be set suitably according to the starting raw materials to be used, the type of target reaction products, and the like.

With the method of the present invention, since the synthesis of halogen-substituted saccharide conventionally carried out in the presence of a catalyst can be performed continuously at high-speed, and, moreover, with no catalyst, a process requiring a long time can be streamlined. In addition, with the method of the present invention, since a catalyst, which is used conventionally, is not used at all, there is no need for post-processing/disposal of the solution after the reaction, such as treatment to neutralize or treatment to render harmless, allowing environmental load reduction to be achieved.

Further, since there is only a still-standing separation operation after the reaction, there is no need for the separation and recovery of a catalyst or an aprotic organic solvent, such that product separation becomes easy. According to the present invention, it is possible to synthesize a halogen-substituted saccharide without using an aprotic solvent, with no catalyst, in a short time on the order of 10 seconds, and with a total yield of 70% or greater. The synthesis method of the present invention is useful as one that can be used for a medicinal product, or the like, allowing a halogen-substituted saccharide to be produced efficiently, in a large amount, at high-speed, and continuously.

In prior art, there has been no example establishing the selective synthesis of a halogen-substituted saccharide while reducing the amount of energy consumption and the amount of waste, and the environmentally friendly type of selective synthesis reaction method for the halogen-substituted saccharide, which is a subject of the present invention, is one which validity has been established for the first time by the present inventors. Moreover, while a halogen-substituted saccharide synthesized from a halide salt and a leaving group-substituted saccharide had the problem that the catalyst and the aprotic organic solvent were remaining with prior art methods, a reaction composition synthesized from a leaving group-substituted saccharide with the present invention has no remaining catalyst and aprotic organic solvent, such that the halogen-substituted saccharide composition of the present invention has an advantage that is not present in prior art products. Consequently, a high safety is meant for the above-mentioned reaction composition in the use as a medicinal product to be ingested by a human.

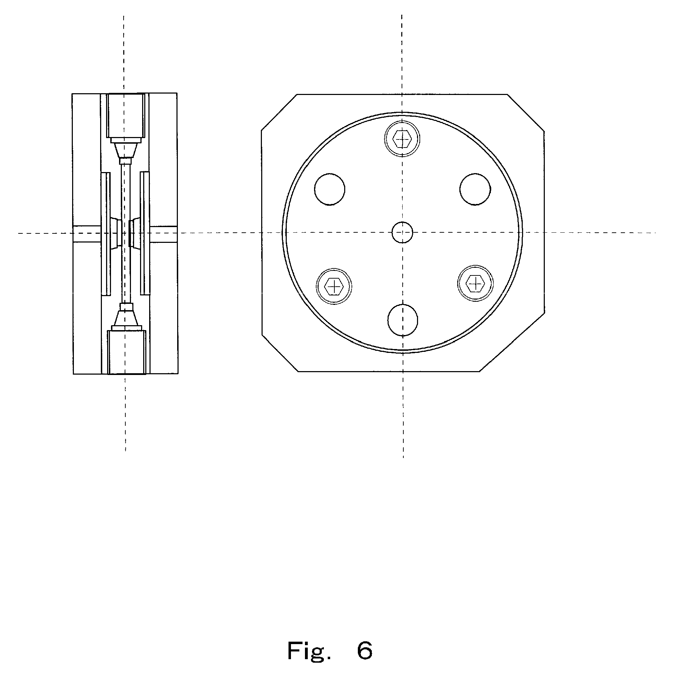

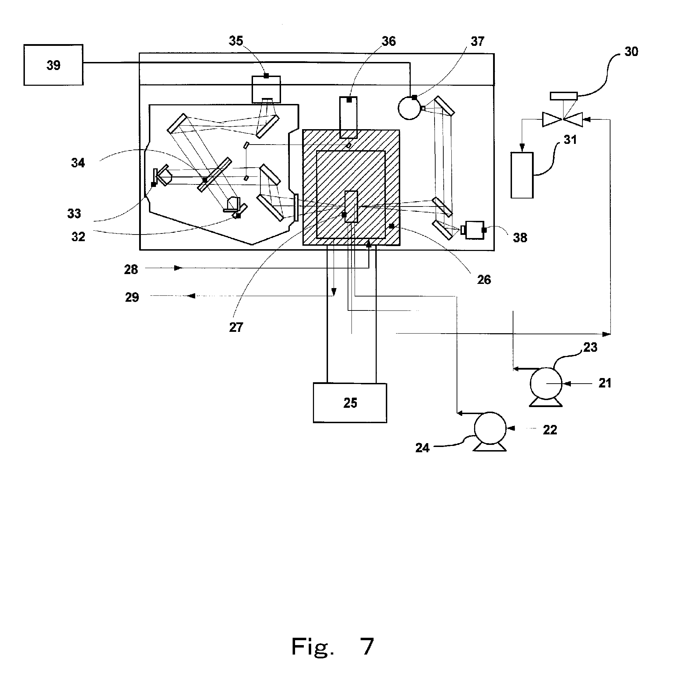

In the present invention, in order to realize a synthesis reaction in catalyst-free condition, it is also possible, for instance, to send a solution comprising a substrate dissolved beforehand in a solvent and use a circulating-type high-temperature and high-pressure infrared spectrophoto in-situ meter (Fig. 7) for observing, by infrared spectroscopy with a high-temperature and high-pressure infrared flow-cell (Fig. 6), the reaction course in the subcritical fluid or the supercritical fluid.

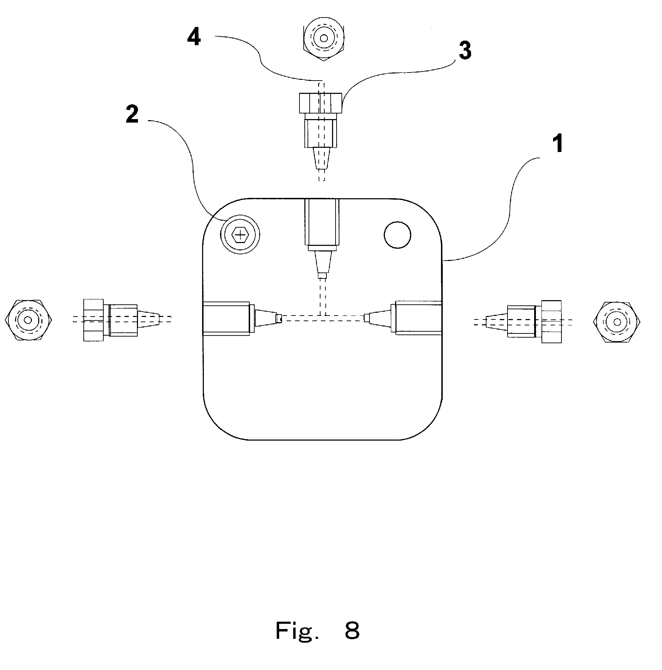

However, exchanging the high temperature and high pressure infrared flow-cell with a windowless high temperature and high pressure flow-cell (Fig. 8), and configuring the piping in such a way that the flow of reactants contact-react directly against the flow of supercritical fluid, allows the synthesis to be performed at a higher flow rate and shorter time, without problems such as leakage occurring in the vicinity of the cell window in the high temperature and high pressure infrared flow-cell. From these, in the examples described below, a device equipped with this windowless high temperature and high pressure flow-cell was used.

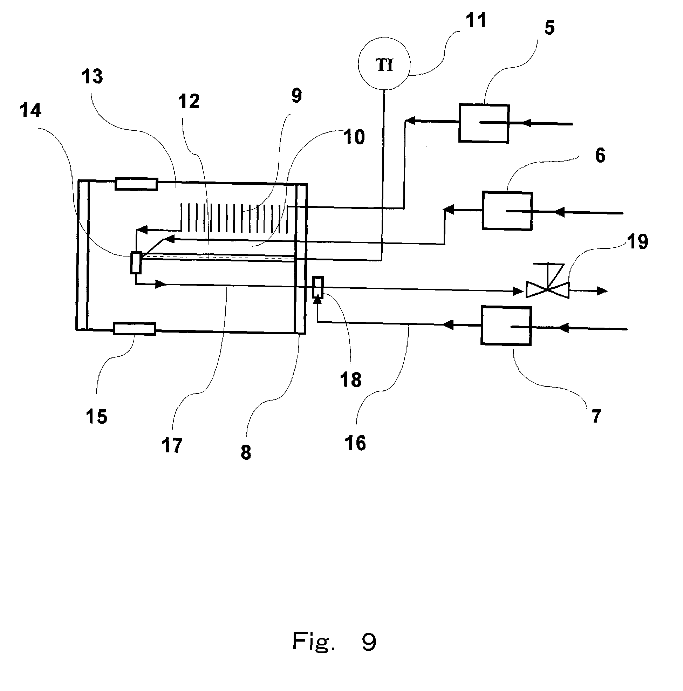

Here, a windowless high-temperature and high-pressure flow-cell main body (Fig. 8) implies, for instance, a screw hole is created in a tee 1 made of commercial SUS316 so that immobilization is possible on a temperature sensor sheath (12 in Fig. 9) described later. The temperature sensor position is adjusted so as to show the cell temperature without measuring the temperature of the furnace body atmosphere and screwed with a sheath immobilization screw and a male screw 2. A piping 4 of SUS316 is connected to tee 1 with a one-ring ferrule-fitted taper screw 3. Obviously, if there are more flow-paths, this windowless cell site is formed not with a tee, but a cross.

Fig. 9 is a furnace body portion of a circulating-type high temperature and high pressure reaction device fitted with a windowless high temperature and high pressure flow-cell which is the reaction device main body. If this is placed inside the hatched position of the circulating-type high temperature and high pressure liquid in-situ infrared spectrophotometer of Fig. 7, while infrared spectra cannot be measured, it can be used as a subcritical/supercritical fluid contact-type synthesis reaction device with variable temperature, pressure and flow rate. Note that the reaction observation in this case is carried out by collecting the aqueous solution after discharge, performing quantification by GC-FID from a calibration curve using a pure product of the product, and performing qualitative analysis by GC/MS.

Hereinafter, explaining the circulating-type high temperature and high pressure reaction device of Fig. 9, a water solution is sent from a water solution-sending pump 5, passes though a cooling flange 8 and then is sent to a furnace body 13. After passage through a tube-coil 9, [the solution] is introduced into a high temperature and high pressure flow-cell 14 supported by and immobilized on a temperature sensor sheath 12 into which a temperature sensor 11 has been inserted in a high temperature and high pressure state.

Meanwhile, a reactant is sent from a reactant solution-sending pump 6, passes through the cooling flange 8 and then is sent to the furnace body 13. After passage through a reactant inlet tube 10, [the solution] is introduced into the high temperature and high pressure flow-cell 14 immobilized on the temperature sensor sheath 12. In addition, washing water solution is sent from pump 7, and after passage through a piping 16, is introduced into a tee 18 to be used for washing.

The solution that has passed through the high temperature and high pressure flow-cell, after passage through a piping 17, passes through the cooling flange 8, and passes outside the furnace body while being air-cooled. Thereafter, discharge solution is collected from a back-pressure valve 19, which sets the pressure, to serve as a sample. Here, if an influence due to heating of the discharge solution containing reactants and products is to be eliminated, it is desirable that a rapid temperature increase is performed and that piping for the reactant inlet line 10 and the discharge solution line 17 is as short as possible, and the water heating coil 9 as long as possible. The present invention is not limited to these; a reaction device can be used similarly if it has similar effects as these.

### Effect of Invention

The following effects are accomplished by the present invention:
(1) a halogen-substituted saccharide can be synthesized from a leaving group-substituted saccharide at a high-speed and continuously.
(2) a halogen-substituted saccharide can be synthesized with high efficiency and selectively, while reducing the amount of waste.
(3) a synthesis process that uses no catalyst and no aprotic organic solvent can be realized.
(4) therefore, a halogen-substituted saccharide composition can be provided which has no catalyst and no aprotic organic solvent remaining within, is not harmful to ecosystems and highly safe for an organism.
(5) if the product does not dissolve in water, a highly pure product can be recovered readily by further pouring water onto the discharged aqueous solution of solid-liquid dispersion, and separating the solution into solid-liquid bilayer while washing.
(6) a novel production technique, which may substitute to existing production process, can be provided as a novel mass production process for a halogen-substituted saccharide useful as a medicinal product.
(7) a simple production technique and a compact production device can be provided, allowing a tracer (several minutes life span) halogen-substituted saccharide composition, which is a radionuclide in positron emission tomography (PET), to be produced within the life span, in small amounts and at high-speed.
(8) since the compounds involved in tracer synthesis are reduced, radioactive contaminated waste can be further reduced.

### BRIEF DESCRIPTION OF THE DRAWINGS

Fig. 1 shows the synthesis of halogen-substituted saccharide from a leaving group-substituted saccharide and a halide salt.
Fig. 2 shows the synthesis pathway of a halogen-substituted saccharide from a leaving group-substituted saccharide and a halide salt.
Fig. 3 shows a synthesis flow chart for a halogen-substituted saccharide using a catalyst/aprotic organic solvent.
Fig. 4 shows a post-processing flow chart for a halogen-substituted saccharide using a catalyst/aprotic organic solvent.
Fig. 5 shows a post-processing flow chart for a halogen-substituted saccharide using a catalyst-free/water solvent.
Fig. 6 shows a high temperature and high pressure infrared flow-cell.
Fig. 7 shows a circulating-type high temperature and high pressure liquid in-situ infrared spectrophotometer used in the samples.
Fig. 8 shows a windowless high temperature and high pressure flow-cell.
Fig. 9 shows the main parts of the circulating-type high temperature and high pressure reaction device used in the examples.
Fig. 10 shows the temperature dependency of pyrans and fluorine-substituted compounds generation in the fluorine-substituted saccharide synthesis from substrates dissolved in an aqueous solution of ethanol (EtOH/H₂O = 95/5) according to the present invention.
Fig. 11 shows the breakdown and the temperature dependency of pyran and fluorine-substituted compounds generation in the fluorine-substituted saccharide synthesis from substrates dissolved in an aqueous solution of ethanol (EtOH/H₂O = 95/5) according to the present invention.
Fig. 12 shows the effects of fluoride agents (potassium fluoride and sodium fluoride) and temperature in the fluorine-substituted saccharide synthesis from substrates dissolved in an aqueous solution of ethanol (EtOH/H₂O = 95/5) according to the present invention.
Fig. 13 shows the effects of the amount of water added in the aqueous solution of ethanol in the fluorine-substituted saccharide synthesis from substrates dissolved in an aqueous solution of ethanol according to the present invention.
Fig. 14 shows the temperature dependency in the fluorine-substituted saccharide synthesis from a fluoride agent (potassium fluoride) and a substrate dissolved in an aqueous solution of ethanol (EtOH/H₂O = 50/50) according to the present invention.
Fig. 15 shows the effects of fluoride agent (potassium fluoride and sodium fluoride) and temperature in the fluorine-substituted saccharide synthesis from a substrate dissolved in an aqueous solution of ethanol (EtOH/H₂O = 50/50) according to the invention.
Fig. 16 effects of sodium hydrogen carbonate in the fluorine-substituted saccharide synthesis from substrates and sodium fluoride of 0.69 equivalents dissolved in an aqueous solution of ethanol (EtOH/H₂O = 50/50)according to the present invention ([a]: TATM-based yield; [b]: NaF-based yield).
Fig. 17 shows the effects of halide agents (sodium fluoride, sodium chloride, sodium bromide and sodium iodide) in the halogen-substituted saccharide synthesis from substrates dissolved in an aqueous solution of ethanol (EtOH/H₂O = 50/50) according to the present invention.

### BEST MODE FOR CARRYING OUT THE INVENTION

In the following, the present invention will be described concretely based on examples; however the present invention is not to be limited in any way by the following examples.

### (Implementation Methods)

First, implementation methods of the present invention will be indicated, and then the examples will be indicated. In the following examples the present invention was carried out using the circulating-type high temperature and high pressure reaction device of Fig. 9, with the synthesis conditions: no catalyst, a temperature of 150 to 300°C, a pressure of 5 to 10 Mpa and a retention time of 3 to 180 seconds. With a device comprising the circulating-type high temperature and high pressure liquid in-situ infrared spectrophotometer of Fig. 7 fitted with the main body (major parts) of the circulating-type high temperature and high pressure reaction device of Fig. 9, first, a prescribed temperature and a prescribed pressure were set, and pure water was sent to a windowless cell (Tee 1) by a pump 5 at a flow rate of 5.0 ml/min.

Thereafter, in an aqueous solution of ethanol prepared beforehand at a proportion of 5 g water to 95 g ethanol, 0.2 g (0.04 mmol) of the substrate β-tetraacetyl-2-deoxy-mannose triflate (β-TATM; in formula Chem. 1, R1, R5, R7 and R10 represent acetoxy groups, R2, R4, R6, R8 and R9 represent hydrogen atoms and L represents OTf group) and 0.02 g (0.04 mmol) of potassium fluoride (in formula Chem. 3, M represents a potassium atom and X represents a fluorine atom) were dissolved, toluene was added as an internal standard (5 mol% of the substrate), and 0.693 ml/min of the mixed solution was sent to the windowless cell (Tee 1) with a pump 6 (aqueous solution concentration after mixing: 0.82 mmol/kg).

After sending the substrate solution, 1 ml of discharge aqueous solution was collected from a back-pressure valve 20 minutes later. When the piping inner volume from the heating furnace to the back-pressure valve outlet was taken as the reaction volume, the reaction time was 10 seconds. To 1 ml of the aqueous solution recovered, 1 ml of acetone was added, [the solution was] shaken, compositional analysis was carried out with a GC/MS analyzer (HP6890, manufactured by Hewlett Packard; column: HP-5; injection port temperature: 250°C; initial column temperature: 75°C (retention time: 0.5 minutes); temperature rise speed: 60°C/minute; final column temperature: 250°C (retention time: 2 minutes)), and the obtained mass spectrum was identified with a matching degree of 90% or greater with the Willey database.

In addition, quantitative determination and, if a commercial reagent exists, qualitative determination were carried out by GC-FID (GC6890, manufactured by Agilent; column: HP-5; injection port temperature: 250°C; split ratio: 5.61; initial column temperature: 75°C (retention time: 0.5 minutes); temperature rise speed: 60°C/minute; final column temperature: 250°C (retention time: 2 minutes)) with toluene as an internal standard.

### Example 1

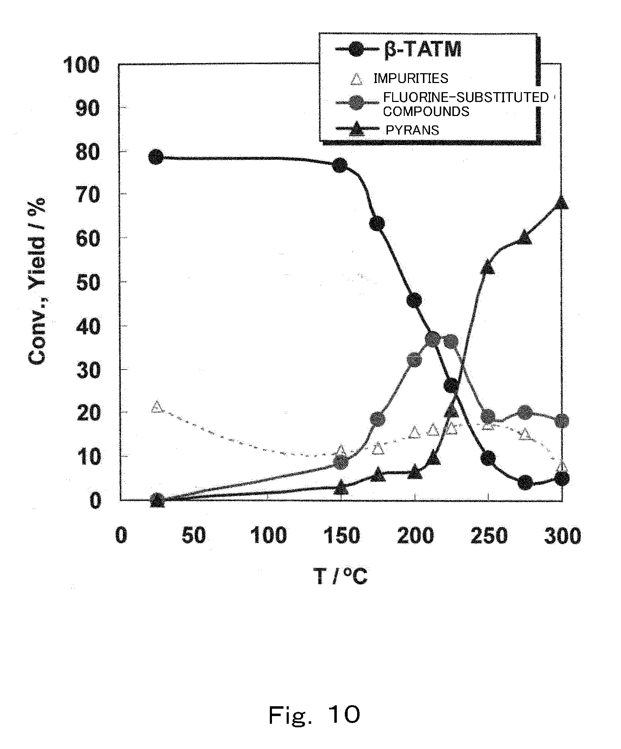

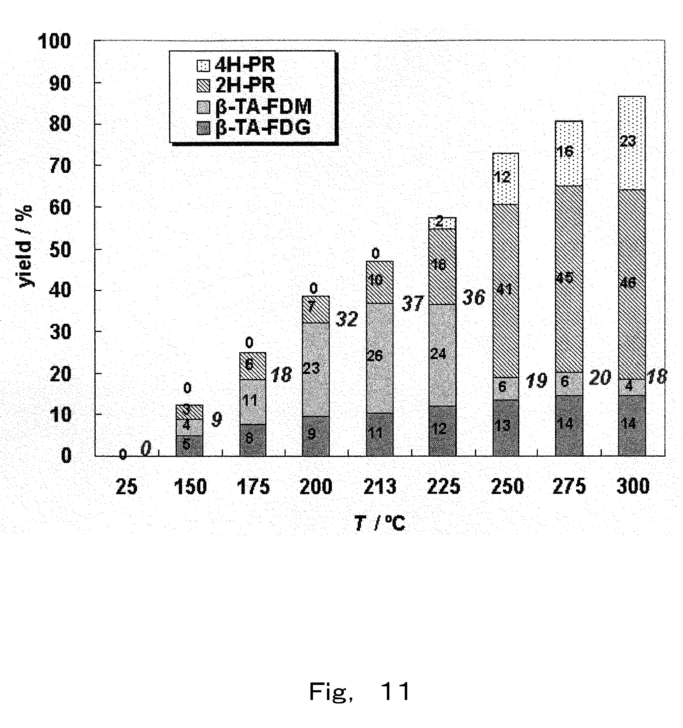

In the above implementation method, when a temperature of 150°C and a pressure of 5 MPa were set, fluorine-substituted compounds were obtained with a total yield of 9%, and pyran compounds with a total yield of 3% (Fig. 10). Here, the breakdown thereof was, as shown in Fig. 11, β-tetraacetyl-2-deoxy-2-fluoroglucose (β-TA-FDG of Fig. 11; in formula Chem. 4, R1, R5, R7 and R10 represent acetoxy groups, R2, R4, R6, R8 and R9 represent hydrogen atoms and X represents a fluorine atom) at a yield of 5%, tetraacetyl-4,5 dihydro-2H-pyran (2H-PR of Fig. 11; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 11; in formula Chem. 7, R1, R6, R7 and R10 represent acetoxy groups, R2, R4, R8 and R9 represent hydrogen atoms) at a yield of 3% and β-tetraacetyl-2-deoxy-2-fluoromannose (β-TA-FDM; in formula Chem. 5, R1, R5, R7 and R10 represent acetoxy groups, R2, R4, R6, R8 and R9 represent hydrogen atoms and X represents a fluorine atom) at a yield of 4%.

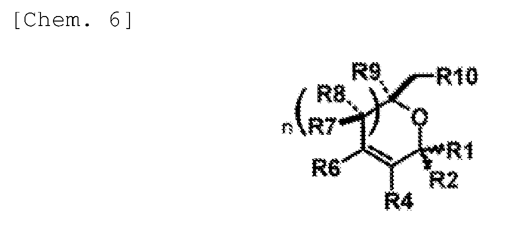

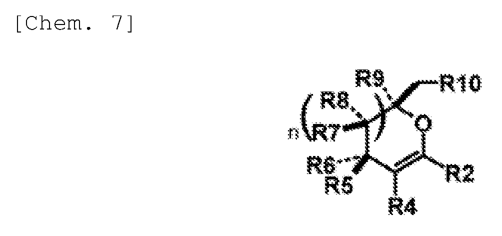

### Example 2

In the above implementation method, when a temperature of 175°C and a pressure of 5 MPa were set, fluorine-substituted compounds were obtained with a total yield of 19%, and pyran compounds with a total yield of 6% (Fig. 10). Here, the breakdown thereof was, as shown in Fig. 11, β-tetraacetyl-2-deoxy-2-fluoroglucose (β-TA-FDG of Fig. 11; in formula Chem. 4, R1, R5, R7 and R10 represent acetoxy groups, R2, R4, R6, R8 and R9 represent hydrogen atoms and X represents a fluorine atom) at a yield of 8%, tetraacetyl-4,5 dihydro-2H-pyran (2H-PR of Fig. 11; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 11; in formula Chem. 7, R1, R6, R7 and R10 represent acetoxy groups, R2, R4, R8 and R9 represent hydrogen atoms) at a yield of 6% and β-tetraacetyl-2-deoxy-2-fluoromannose (β-TA-FDM of Fig. 11; in formula Chem. 5, R1, R5, R7 and R10 represent acetoxy groups, R2, R4, R6, R8 and R9 represent hydrogen atoms and X represents a fluorine atom) at a yield of 11%.

### Example 3

In the above implementation method, when a temperature of 200°C and a pressure of 5 MPa were set, fluorine-substituted compounds were obtained with a total yield of 32%, and pyran compounds with a total yield of 7% (Fig. 10). Here, the breakdown thereof was, as shown in Fig. 11, β-tetraacetyl-2-deoxy-2-fluoroglucose (β-TA-FDG of Fig. 11; in formula Chem. 4, R1, R5, R7 and R10 represent acetoxy groups, R2, R4, R6, R8 and R9 represent hydrogen atoms and X represents a fluorine atom) at a yield of 9%, tetraacetyl-4,5 dihydro-2H-pyran (2H-PR of Fig. 11; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 11; in formula Chem. 7, R1, R6, R7 and R10 represent acetoxy groups, R2, R4, R8 and R9 represent hydrogen atoms) at a yield of 7% and β-tetraacetyl-2-deoxy-2-fluoromannose (β-TA-FDM of Fig. 11; in formula Chem. 5, R1, R5, R7 and R10 represent acetoxy groups, R2, R4, R6, R8 and R9 represent hydrogen atoms and X represents a fluorine atom) at a yield of 23%.

### Example 4

In the above implementation method, when a temperature of 213°C and a pressure of 5 MPa were set, fluorine-substituted compounds were obtained with a total yield of 37%, and pyran compounds with a total yield of 10% (Fig. 10). Here, the breakdown thereof was, as shown in Fig. 11, β-tetraacetyl-2-deoxy-2-fluoroglucose (β-TA-FDG of Fig. 11; in formula Chem. 4, R1, R5, R7 and R10 represent acetoxy groups, R2, R4, R6, R8 and R9 represent hydrogen atoms and X represents a fluorine atom) at a yield of 11%, tetraacetyl-4,5 dihydro-2H-pyran (2H-PR of Fig. 11; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 11; in formula Chem. 7, R1, R6, R7 and R10 represent acetoxy groups, R2, R4, R8 and R9 represent hydrogen atoms) at a yield of 10% and β-tetraacetyl-2-deoxy-2-fluoromannose (β-TA-FDM of Fig. 11; in formula Chem. 5, R1, R5, R7 and R10 represent acetoxy groups, R2, R4, R6, R8 and R9 represent hydrogen atoms and X represents a fluorine atom) at a yield of 26%.

### Example 5

In the above implementation method, when a temperature of 225°C and a pressure of 5 MPa were set, fluorine-substituted compounds were obtained with a total yield of 36%, and pyran compounds with a total yield of 20% (Fig. 10). Here, the breakdown thereof was, as shown in Fig. 11, β-tetraacetyl-2-deoxy-2-fluoroglucose (β-TA-FDG of Fig. 11; in formula Chem. 4, R1, R5, R7 and R10 represent acetoxy groups, R2, R4, R6, R8 and R9 represent hydrogen atoms and X represents a fluorine atom) at a yield of 12%, tetraacetyl-4,5 dihydro-2H-pyran (2H-PR of Fig. 11; in formula Chem. 6, R1, R6, R7 and R10 represent acetoxy groups, R2, R4, R8 and R9 represent hydrogen atoms) at a yield of 2%, tetraacetyl-4,5 dihydro-4H-pyran (4H-PR of Fig. 11; in formula Chem. 7, R1, R6, R7 and R10 represent acetoxy groups, R2, R4, R8 and R9 represent hydrogen atoms) at a yield of 18% and β-tetraacetyl-2-deoxy-2-fluoromannose (β-TA-FDM of Fig. 11; in formula Chem. 5, R1, R5, R7 and R10 represent acetoxy groups, R2, R4, R6, R8 and R9 represent hydrogen atoms and X represents a fluorine atom) at a yield of 24%.

### Example 6

In the above implementation method, when a temperature of 250°C and a pressure of 5 MPa were set, fluorine-substituted compounds were obtained with a total yield of 19%, and pyran compounds with a total yield of 53% (Fig. 10). Here, the breakdown thereof was, as shown in Fig. 11, β-tetraacetyl-2-deoxy-2-fluoroglucose (β-TA-FDG of Fig. 11; in formula Chem. 4, R1, R5, R7 and R10 represent acetoxy groups, R2, R4, R6, R8 and R9 represent hydrogen atoms and X represents a fluorine atom) at a yield of 13%, tetraacetyl-4,5 dihydro-2H-pyran (2H-PR of Fig. 11; in formula Chem. 6, R1, R6, R7 and R10 represent acetoxy groups, R2, R4, R8 and R9 represent hydrogen atoms) at a yield of 12%, tetraacetyl-4,5 dihydro-4H-pyran (4H-PR of Fig. 11; in formula Chem. 7, R1, R6, R7 and R10 represent acetoxy groups, R2, R4, R8 and R9 represent hydrogen atoms) at a yield of 41% and β-tetraacetyl-2-deoxy-2-fluoromannose (β-TA-FDM of Fig. 11; in formula Chem. 5, R1, R5, R7 and R10 represent acetoxy groups, R2, R4, R6, R8 and R9 represent hydrogen atoms and X represents a fluorine atom) at a yield of 6%.

### Example 7

In the above implementation method, when a temperature of 275°C and a pressure of 6 MPa were set, fluorine-substituted compounds were obtained with a total yield of 20%, and pyran compounds with a total yield of 61% (Fig. 10). Here, the breakdown thereof was, as shown in Fig. 11, β-tetraacetyl-2-deoxy-2-fluoroglucose (β-TA-FDG of Fig. 11; in formula Chem. 4, R1, R5, R7 and R10 represent acetoxy groups, R2, R4, R6, R8 and R9 represent hydrogen atoms and X represents a fluorine atom) at a yield of 14%, tetraacetyl-4,5 dihydro-2H-pyran (2H-PR of Fig. 11; in formula Chem. 6, R1, R6, R7 and R10 represent acetoxy groups, R2, R4, R8 and R9 represent hydrogen atoms) at a yield of 16%, tetraacetyl-4,5 dihydro-4H-pyran (4H-PR of Fig. 11; in formula Chem. 7, R1, R6, R7 and R10 represent acetoxy groups, R2, R4, R8 and R9 represent hydrogen atoms) at a yield of 45% and β-tetraacetyl-2-deoxy-2-fluoromannose (β-TA-FDM of Fig. 11; in formula Chem. 5, R1, R5, R7 and R10 represent acetoxy groups, R2, R4, R6, R8 and R9 represent hydrogen atoms and X represents a fluorine atom) at a yield of 6%.

### Example 8

In the above implementation method, when a temperature of 300°C and a pressure of 10 MPa were set, fluorine-substituted compounds were obtained with a total yield of 18%, and pyran compounds with a total yield of 69% (Fig. 10). Here, the breakdown thereof was, as shown in Fig. 11, β-tetraacetyl-2-deoxy-2-fluoroglucose (β-TA-FDG of Fig. 11; in formula Chem. 4, R1, R5, R7 and R10 represent acetoxy groups, R2, R4, R6, R8 and R9 represent hydrogen atoms and X represents a fluorine atom) at a yield of 14%, tetraacetyl-4,5 dihydro-2H-pyran (2H-PR of Fig. 11; in formula Chem. 6, R1, R6, R7 and R10 represent acetoxy groups, R2, R4, R8 and R9 represent hydrogen atoms) at a yield of 23%, tetraacetyl-4,5 dihydro-4H-pyran (4H-PR of Fig. 11; in formula Chem. 7, R1, R6, R7 and R10 represent acetoxy groups, R2, R4, R8 and R9 represent hydrogen atoms) at a yield of 46% and β-tetraacetyl-2-deoxy-2-fluoromannose (β-TA-FDM of Fig. 11; in formula Chem. 5, R1, R5, R7 and R10 represent acetoxy groups, R2, R4, R6, R8 and R9 represent hydrogen atoms and X represents a fluorine atom) at a yield of 4%.

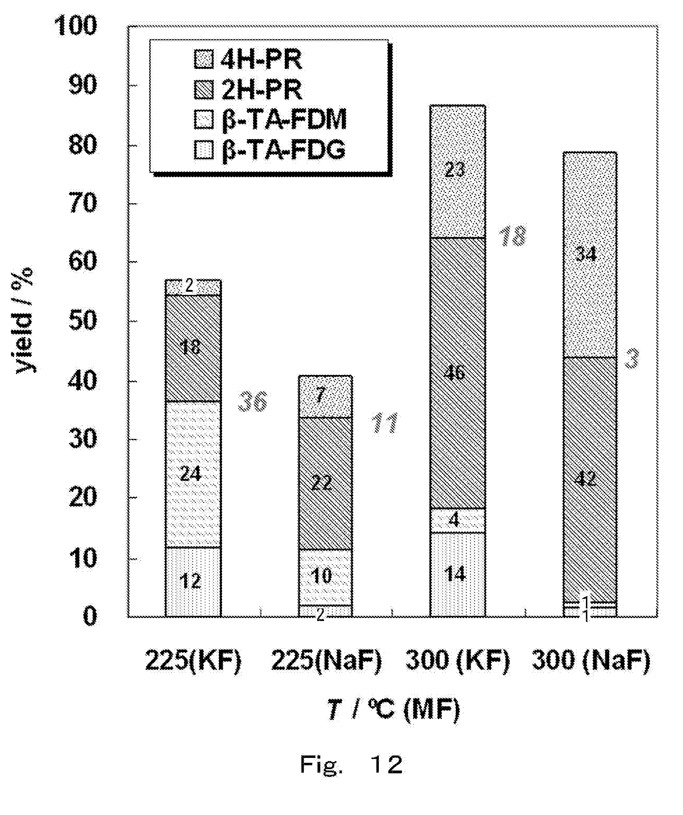

Meanwhile, when [the method] was carried out using, instead of potassium fluoride (in formula Chem. 3, M represents a potassium atom and X represents a fluorine atom), sodium fluoride (in formula Chem. 3, M represents a sodium atom and X represents a fluorine atom), which reagent per se can be used as a PET reagent for bones, the results of Examples 9 and 10 described below were obtained. As shown in Fig. 12, when sodium fluoride (NaF) and potassium fluoride (KF) were compared, it was shown that, in the case of sodium fluoride, reactivity was lower than in the case of potassium fluoride.

### Example 9

In the above implementation method, when a temperature of 225°C and a pressure of 5 MPa were set, fluorine-substituted compounds were obtained with a total yield of 11%, and pyran compounds with a total yield of 29% (Fig. 12). Here, the breakdown thereof was, as shown in Fig. 12, β-tetraacetyl-2-deoxy-2-fluoroglucose (β-TA-FDG of Fig. 12; in formula Chem. 4, R1, R5, R7 and R10 represent acetoxy groups, R2, R4, R6, R8 and R9 represent hydrogen atoms and X represents a fluorine atom) at a yield of 2%, tetraacetyl-4,5 dihydro-2H-pyran (2H-PR of Fig. 12; in formula Chem. 6, R1, R6, R7 and R10 represent acetoxy groups, R2, R4, R8 and R9 represent hydrogen atoms) at a yield of 22%, tetraacetyl-4,5 dihydro-4H-pyran (4H-PR of Fig. 12; in formula Chem. 7, R1, R6, R7 and R10 represent acetoxy groups, R2, R4, R8 and R9 represent hydrogen atoms) at a yield of 7% and β-tetraacetyl-2-deoxy-2-fluoromannose (β-TA-FDM of Fig. 12; in formula Chem. 5, R1, R5, R7 and R10 represent acetoxy groups, R2, R4, R6, R8 and R9 represent hydrogen atoms and X represents a fluorine atom) at a yield of 10%.

### Example 10

In the above implementation method, when a temperature of 300°C and a pressure of 5 MPa were set, fluorine-substituted compounds were obtained with a total yield of 11%, and pyran compounds with a total yield of 29% (Fig. 12). Here, the breakdown thereof was, as shown in Fig. 12, β-tetraacetyl-2-deoxy-2-fluoroglucose (β-TA-FDG of Fig. 12; in formula Chem. 4, R1, R5, R7 and R10 represent acetoxy groups, R2, R4, R6, R8 and R9 represent hydrogen atoms and X represents a fluorine atom) at a yield of 2%, tetraacetyl-4,5 dihydro-2H-pyran (2H-PR of Fig. 12; in formula Chem. 6, R1, R6, R7 and R10 represent acetoxy groups, R2, R4, R8 and R9 represent hydrogen atoms) at a yield of 42%, tetraacetyl-4,5 dihydro-4H-pyran (4H-PR of Fig. 12; in formula Chem. 7, R1, R6, R7 and R10 represent acetoxy groups, R2, R4, R8 and R9 represent hydrogen atoms) at a yield of 34% and β-tetraacetyl-2-deoxy-2-fluoromannose (β-TA-FDM of Fig. 12; in formula Chem. 5, R1, R5, R7 and R10 represent acetoxy groups, R2, R4, R6, R8 and R9 represent hydrogen atoms and X represents a fluorine atom) at a yield of 1%.

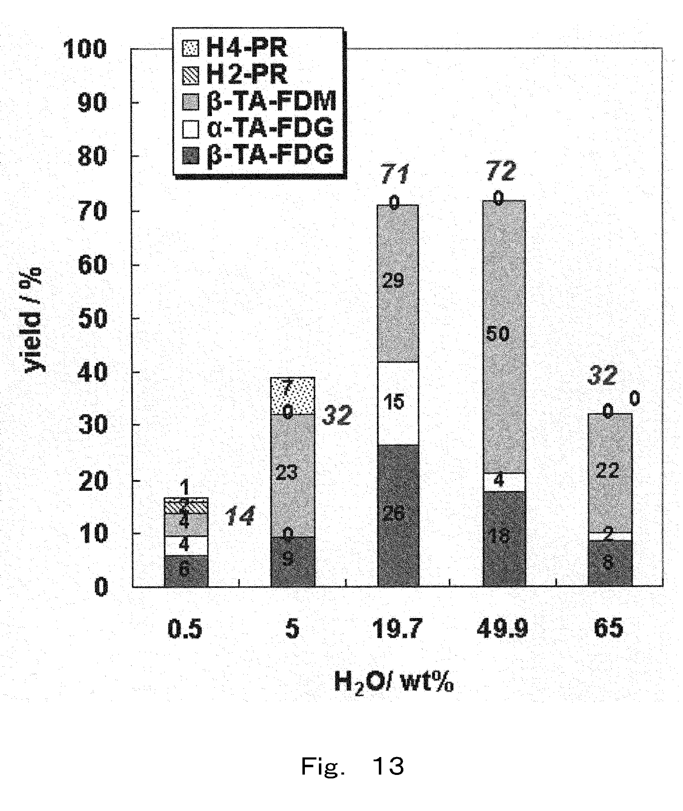

Here, from the fact that the yield was low for the fluorine-substituted compounds, when [the method] was carried out by altering the proportion of water in the ethanol/water solvent that dissolves β-tetraacetyl-2-deoxy-mannose triflate (β-TATM; in formula Chem. 1, R1, R5, R7 and R10 represent acetoxy groups, R2, R4, R6, R8 and R9 represent hydrogen atoms and L represents OTf group) and using potassium fluoride (in formula Chem. 3, M represents a potassium atom and X represents a fluorine atom), which reagent per se can be used as a PET reagent for bones, as the fluoride agent, the results shown in the example below and in Fig. 13 were obtained. From these results, as with ethanol/water = 50/50 (weight ratio), when an aqueous solution of ethanol containing water to some extent was used as a solvent for β-TATM, generation of pyrans was suppressed and generation of fluorine-substituted compounds increased considerably.

### Example 11

In the above implementation method, when the raw material β-TATM was dissolved in a solvent of ethanol/water = 99.5/0.5 (weight ratio), a temperature of 200°C and a pressure of 5 MPa were set, fluorine-substituted compounds were obtained with a total yield of 14%, and pyran compounds with a total yield of 3% (Fig. 13). Here, the breakdown thereof was, as shown in Fig. 13, β-tetraacetyl-2-deoxy-2-fluoroglucose (β-TA-FDG of Fig. 13; in formula Chem. 4, R1, R5, R7 and R10 represent acetoxy groups, R2, R4, R6, R8 and R9 represent hydrogen atoms and X represents a fluorine atom) at a yield of 2%, tetraacetyl-4,5 dihydro-2H-pyran (2H-PR of Fig. 13; in formula Chem. 6, R1, R6, R7 and R10 represent acetoxy groups, R2, R4, R8 and R9 represent hydrogen atoms) at a yield of 2%, tetraacetyl-4,5 dihydro-4H-pyran (4H-PR of Fig. 13; in formula Chem. 7, R1, R6, R7 and R10 represent acetoxy groups, R2, R4, R8 and R9 represent hydrogen atoms) at a yield of 1%, α-tetraacetyl-2-deoxy-2-fluoroglucose (α-TA-FDG of Fig. 13; in formula Chem. 4, R1, R5, R7 and R10 represent acetoxy groups, R2, R4, R6, R8 and R9 represent hydrogen atoms and X represents a fluorine atom) at a yield of 2% and β-tetraacetyl-2-deoxy-2-fluoromannose (β-TA-FDM of Fig. 13; in formula Chem. 5, R1, R5, R7 and R10 represent acetoxy groups, R2, R4, R6, R8 and R9 represent hydrogen atoms and X represents a fluorine atom) at a yield of 4%.

### Example 12

In the above implementation method, when the raw material β-TATM was dissolved in a solvent of ethanol/water = 95/5 (weight ratio) and a temperature of 200°C and a pressure of 5 MPa were set, fluorine-substituted compounds were obtained with a total yield of 32%, and pyran compounds with a total yield of 7% (Fig. 13). Here, the breakdown thereof was, as shown in Fig. 13, β-tetraacetyl-2-deoxy-2-fluoroglucose (β-TA-FDG of Fig. 13; in formula Chem. 4, R1, R5, R7 and R10 represent acetoxy groups, R2, R4, R6, R8 and R9 represent hydrogen atoms and X represents a fluorine atom) at a yield of 9%, tetraacetyl-4,5 dihydro-2H-pyran (2H-PR of Fig. 13; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 13; in formula Chem. 7, R1, R6, R7 and R10 represent acetoxy groups, R2, R4, R8 and R9 represent hydrogen atoms) at a yield of 7%, α-tetraacetyl-2-deoxy-2-fluoroglucose (α-TA-FDG of Fig. 13; in formula Chem. 4, R1, R5, R7 and R10 represent acetoxy groups, R2, R4, R6, R8 and R9 represent hydrogen atoms and X represents a fluorine atom) at a yield of 0% and β-tetraacetyl-2-deoxy-2-fluoromannose (β-TA-FDM of Fig. 13; in formula Chem. 5, R1, R5, R7 and R10 represent acetoxy groups, R2, R4, R6, R8 and R9 represent hydrogen atoms and X represents a fluorine atom) at a yield of 23%.

### Example 13

In the above implementation method, when the raw material β-TATM was dissolved in a solvent of ethanol/water = 80/20 (weight ratio) and a temperature of 200°C and a pressure of 5 MPa were set, fluorine-substituted compounds were obtained with a total yield of 71%, and pyran compounds with a total yield of 0% (Fig. 13). Here, the breakdown thereof was, as shown in Fig. 13, β-tetraacetyl-2-deoxy-2-fluoroglucose (β-TA-FDG of Fig. 13; in formula Chem. 4, R1, R5, R7 and R10 represent acetoxy groups, R2, R4, R6, R8 and R9 represent hydrogen atoms and X represents a fluorine atom) at a yield of 26%, tetraacetyl-4,5 dihydro-2H-pyran (2H-PR of Fig. 13; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 13; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 13; in formula Chem. 4, R1, R5, R7 and R10 represent acetoxy groups, R2, R4, R6, R8 and R9 represent hydrogen atoms and X represents a fluorine atom) at a yield of 15% and β-tetraacetyl-2-deoxy-2-fluoromannose (β-TA-FDM of Fig. 13; in formula Chem. 5, R1, R5, R7 and R10 represent acetoxy groups, R2, R4, R6, R8 and R9 represent hydrogen atoms and X represents a fluorine atom) at a yield of 29%.

### Example 14

In the above implementation method, when the raw material β-TATM was dissolved in a solvent of ethanol/water = 50/50 (weight ratio) and a temperature of 200°C and a pressure of 5 MPa were set, fluorine-substituted compounds were obtained with a total yield of 72%, and pyran compounds with a total yield of 0% (Fig. 13). Here, the breakdown thereof was, as shown in Fig. 13, β-tetraacetyl-2-deoxy-2-fluoroglucose (β-TA-FDG of Fig. 13; in formula Chem. 4, R1, R5, R7 and R10 represent acetoxy groups, R2, R4, R6, R8 and R9 represent hydrogen atoms and X represents a fluorine atom) at a yield of 18%, tetraacetyl-4,5 dihydro-2H-pyran (2H-PR of Fig. 13; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 13; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 13; in formula Chem. 4, R1, R5, R7 and R10 represent acetoxy groups, R2, R4, R6, R8 and R9 represent hydrogen atoms and X represents a fluorine atom) at a yield of 4% and β-tetraacetyl-2-deoxy-2-fluoromannose (β-TA-FDM of Fig. 13; in formula Chem. 5, R1, R5, R7 and R10 represent acetoxy groups, R2, R4, R6, R8 and R9 represent hydrogen atoms and X represents a fluorine atom) at a yield of 50%.

### Example 15

In the above implementation method, when the raw material β-TATM was dissolved in a solvent of ethanol/water = 35/65 (weight ratio) and a temperature of 200°C and a pressure of 5 MPa were set, fluorine-substituted compounds were obtained with a total yield of 24%, and pyran compounds with a total yield of 0% (Fig. 13). Here, the breakdown thereof was, as shown in Fig. 13, β-tetraacetyl-2-deoxy-2-fluoroglucose (β-TA-FDG of Fig. 13; in formula Chem. 4, R1, R5, R7 and R10 represent acetoxy groups, R2, R4, R6, R8 and R9 represent hydrogen atoms and X represents a fluorine atom) at a yield of 6%, tetraacetyl-4,5 dihydro-2H-pyran (2H-PR of Fig. 13; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 13; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 13; in formula Chem. 4, R1, R5, R7 and R10 represent acetoxy groups, R2, R4, R6, R8 and R9 represent hydrogen atoms and X represents a fluorine atom) at a yield of 1% and β-tetraacetyl-2-deoxy-2-fluoromannose (β-TA-FDM of Fig. 13; in formula Chem. 5, R1, R5, R7 and R10 represent acetoxy groups, R2, R4, R6, R8 and R9 represent hydrogen atoms and X represents a fluorine atom) at a yield of 16%.

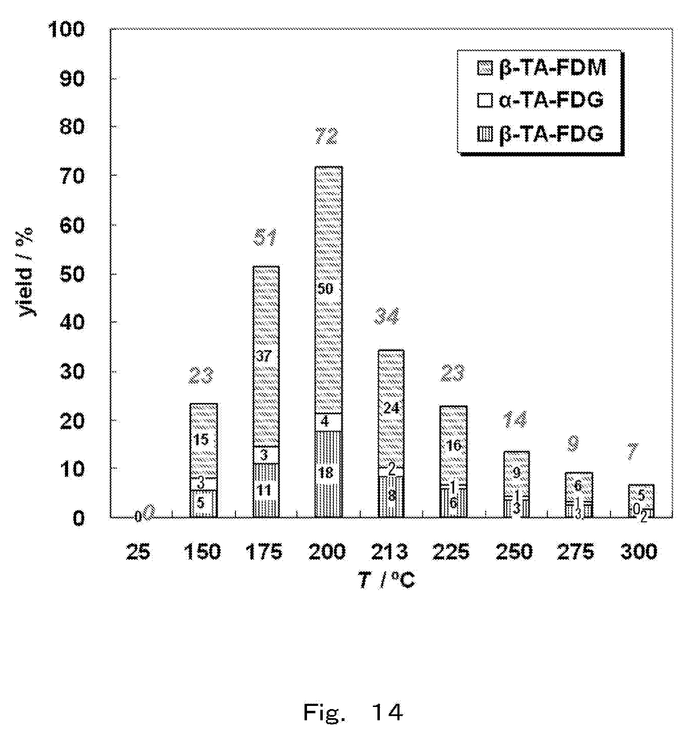

Here, from the fact that the yield was low for the fluorine-substituted compounds, when a method was carried out, with β-tetraacetyl-2-deoxy-mannose triflate (β-TATM; in formula Chem. 1, R1, R5, R7 and R10 represent acetoxy groups, R2, R4, R6, R8 and R9 represent hydrogen atoms and L represents OTf group) being constant at ethanol/water = 50/50 (weight ratio) in an aqueous solution of ethanol, which is the dissolving solvent, potassium fluoride (in formula Chem. 3, M represents a potassium atom and X represents a fluorine atom) was thus used as the fluoride agent, whereby the results shown in the example below and in Fig. 14 were obtained. From these results, as with ethanol/water = 50/50 (weight ratio), when an aqueous solution of ethanol containing water to some extent was used as a solvent for β-TATM, generation of pyrans was suppressed and generation of fluorine-substituted compounds increased considerably.

### Example 16

In the above implementation method, when the raw material β-TATM was dissolved in a solvent of ethanol/water = 50/50 (weight ratio) and a temperature of 25°C and a pressure of 5 MPa were set, fluorine-substituted compounds had a total yield of 0%, and pyran compounds a total yield of 0% (Fig. 14).

### Example 17

In the above implementation method, when the raw material β-TATM was dissolved in a solvent of ethanol/water = 50/50 (weight ratio) and a temperature of 150°C and a pressure of 5 MPa were set, fluorine-substituted compounds were obtained with a total yield of 23%, and pyran compounds with a total yield of 0% (Fig. 14). Here, the breakdown thereof was, as shown in Fig. 14, β-tetraacetyl-2-deoxy-2-fluoroglucose (β-TA-FDC of Fig. 14; in formula Chem. 4, R1, R5, R7 and R10 represent acetoxy groups, R2, R4, R6, R8 and R9 represent hydrogen atoms and X represents a fluorine atom) at a yield of 5%, tetraacetyl-4,5 dihydro-2H-pyran (2H-PR of Fig. 14; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 14; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 3% and β-tetraacetyl-2-deoxy-2-fluoromannose (β-TA-FDM of Fig. 14; in formula Chem. 5, R1, R5, R7 and R10 represent acetoxy groups, R2, R4, R6, R8 and R9 represent hydrogen atoms and X represents a fluorine atom) at a yield of 15%.

### Example 18

In the above implementation method, when the raw material β-TATM was dissolved in a solvent of ethanol/water = 50/50 (weight ratio) and a temperature of 175°C and a pressure of 5 MPa were set, fluorine-substituted compounds were obtained with a total yield of 51%, and pyran compounds with a total yield of 0% (Fig. 14). Here, the breakdown thereof was, as shown in Fig. 14, β-tetraacetyl-2-deoxy-2-fluoroglucose (β-TA-FDC of Fig. 14; in formula Chem. 4, R1, R5, R7 and R10 represent acetoxy groups, R2, R4, R6, R8 and R9 represent hydrogen atoms and X represents a fluorine atom) at a yield of 11%, tetraacetyl-4,5 dihydro-2H-pyran (2H-PR of Fig. 14; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 14; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 3% and β-tetraacetyl-2-deoxy-2-fluoromannose (β-TA-FDM of Fig. 14; in formula Chem. 5, R1, R5, R7 and R10 represent acetoxy groups, R2, R4, R6, R8 and R9 represent hydrogen atoms and X represents a fluorine atom) at a yield of 37%.

### Example 19

In the above implementation method, when the raw material β-TATM was dissolved in a solvent of ethanol/water = 50/50 (weight ratio) and a temperature of 200°C and a pressure of 5 MPa were set, fluorine-substituted compounds were obtained with a total yield of 72%, and pyran compounds with a total yield of 0% (Fig. 14). Here, the breakdown thereof was, as shown in Fig. 14, β-tetraacetyl-2-deoxy-2-fluoroglucose (β-TA-FDC of Fig. 14; in formula Chem. 4, R1, R5, R7 and R10 represent acetoxy groups, R2, R4, R6, R8 and R9 represent hydrogen atoms and X represents a fluorine atom) at a yield of 18%, tetraacetyl-4,5 dihydro-2H-pyran (2H-PR of Fig. 14; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 14; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 4% and β-tetraacetyl-2-deoxy-2-fluoromannose (β-TA-FDM of Fig. 14, in formula Chem. 5, R1, R5, R7 and R10 represent acetoxy groups, R2, R4, R6, R8 and R9 represent hydrogen atoms and X represents a fluorine atom) at a yield of 50%.

### Example 20

In the above implementation method, when the raw material β-TATM was dissolved in a solvent of ethanol/water = 50/50 (weight ratio) and a temperature of 213°C and a pressure of 5 MPa were set, fluorine-substituted compounds were obtained with a total yield of 34%, and pyran compounds with a total yield of 0% (Fig. 14). Here, the breakdown thereof was, as shown in Fig. 14, β-tetraacetyl-2-deoxy-2-fluoroglucose (β-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 8%, tetraacetyl-4,5 dihydro-2H-pyran (2H-PR of Fig. 14; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 14; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 2% and β-tetraacetyl-2-deoxy-2-fluoromannose (β-TA-FDM of Fig. 14; in formula Chem. 5, R1, R5, R7 and R10 represent acetoxy groups, R2, R4, R6, R8 and R9 represent hydrogen atoms and X represents a fluorine atom) at a yield of 24%.

### Example 21

In the above implementation method, when the raw material β-TATM was dissolved in a solvent of ethanol/water = 50/50 (weight ratio) and a temperature of 225°C and a pressure of 5 MPa were set, fluorine-substituted compounds were obtained with a total yield of 23%, and pyran compounds with a total yield of 0% (Fig. 14). Here, the breakdown thereof was, as shown in Fig. 14, β-tetraacetyl-2-deoxy-2-fluoroglucose (β-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 6%, tetraacetyl-4,5 dihydro-2H-pyran (2H-PR of Fig. 14; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 14; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 1% and β-tetraacetyl-2-deoxy-2-fluoromannose (β-TA-FDM of Fig. 14; in formula Chem. 5, R1, R5, R7 and R10 represent acetoxy groups, R2, R4, R6, R8 and R9 represent hydrogen atoms and X represents a fluorine atom) at a yield of 16%.

### Example 22

In the above implementation method, when the raw material β-TATM was dissolved in a solvent of ethanol/water = 50/50 (weight ratio) and a temperature of 250°C and a pressure of 5 MPa were set, fluorine-substituted compounds were obtained with a total yield of 14%, and pyran compounds with a total yield of 0% (Fig. 14). Here, the breakdown thereof was, as shown in Fig. 14, β-tetraacetyl-2-deoxy-2-fluoroglucose (β-TA-FDC of Fig. 14; in formula Chem. 4, R1, R5, R7 and R10 represent acetoxy groups, R2, R4, R6, R8 and R9 represent hydrogen atoms and X represents a fluorine atom) at a yield of 3%, tetraacetyl-4,5 dihydro-2H-pyran (2H-PR of Fig. 14; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 14; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 1% and β-tetraacetyl-2-deoxy-2-fluoromannose (β-TA-FDM of Fig. 14; in formula Chem. 5, R1, R5, R7 and R10 represent acetoxy groups, R2, R4, R6, R8 and R9 represent hydrogen atoms and X represents a fluorine atom) at a yield of 9%.

### Example 23

In the above implementation method, when the raw material β-TATM was dissolved in a solvent of ethanol/water = 50/50 (weight ratio) and a temperature of 275°C and a pressure of 5 MPa were set, fluorine-substituted compounds were obtained with a total yield of 9%, and pyran compounds with a total yield of 0% (Fig. 14). Here, the breakdown thereof was, as shown in Fig. 14, β-tetraacetyl-2-deoxy-2-fluoroglucose (β-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 3%, tetraacetyl-4,5 dihydro-2H-pyran (2H-PR of Fig. 14; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 14; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 6% and β-tetraacetyl-2-deoxy-2-fluoromannose (β-TA-FDM of Fig. 14; in formula Chem. 5, R1, R5, R7 and R10 represent acetoxy groups, R2, R4, R6, R8 and R9 represent hydrogen atoms and X represents a fluorine atom) at a yield of 2%.

### Example 24

In the above implementation method, when the raw material β-TATM was dissolved in a solvent of ethanol/water = 50/50 (weight ratio) and a temperature of 300°C and a pressure of 5 MPa were set, fluorine-substituted compounds were obtained with a total yield of 7%, and pyran compounds with a total yield of 0% (Fig. 14). Here, the breakdown thereof was, as shown in Fig. 14, β-tetraacetyl-2-deoxy-2-fluoroglucose (β-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 2%, tetraacetyl-4,5 dihydro-2H-pyran (2H-PR of Fig. 14; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 14; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 14; in formula Chem. 4, R1, R5, R7 and R10 represent acetoxy groups, R2, R4, R6, R8 and R9 represent hydrogen atoms and X represents a fluorine atom) at a yield of 0% and β-tetraacetyl-2-deoxy-2-fluoromannose (β-TA-FDM of Fig. 14; in formula Chem. 5, R1, R5, R7 and R10 represent acetoxy groups, R2, R4, R6, R8 and R9 represent hydrogen atoms and X represents a fluorine atom) at a yield of 5%.

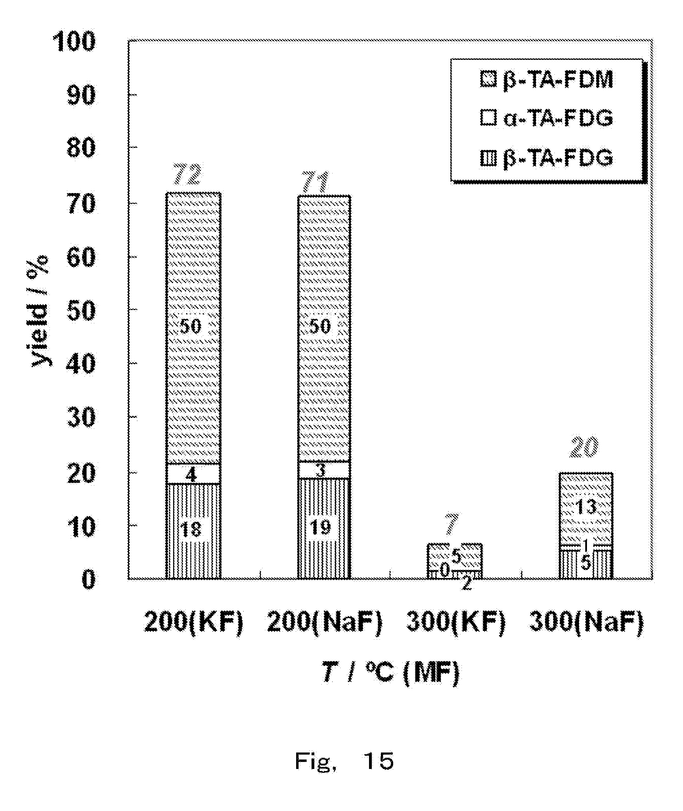

Meanwhile, when [the method] was carried out by dissolving the raw material β-TATM in a solvent of ethanol/water = 50/50 (weight ratio) and using, instead of potassium fluoride (in formula Chem. 3, M represents a potassium atom and X represents a fluorine atom), sodium fluoride (in formula Chem. 3, M represents a sodium atom and X represents a fluorine atom), which reagent per se can be used as a PET reagent for bones, the results of Examples 25 and 26 described below were obtained. As shown in Fig. 15, when sodium fluoride (NaF) and potassium fluoride (KF) were compared, it was found that, when the raw material β-TATM was dissolved in ethanol/water = 50/50 (weight ratio), the results of potassium fluoride and sodium fluoride were approximately on the same order.

### Example 25

In the above implementation method, when a temperature of 200°C and a pressure of 5 MPa were set, fluorine-substituted compounds were obtained with a total yield of 71%, and pyran compounds with a total yield of 0% (Fig. 15). Here, the breakdown thereof was, as shown in Fig. 15, β-tetraacetyl-2-deoxy-2-fluoroglucose (β-TA-FDG of Fig. 15; in formula Chem. 4, R1, R5, R7 and R10 represent acetoxy groups, R2, R4, R6, R8 and R9 represent hydrogen atoms and X represents a fluorine atom) at a yield of 19%, tetraacetyl-4,5 dihydro-2H-pyran (2H-PR of Fig. 15; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 15; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 15; in formula Chem. 4, R1, R5, R7 and R10 represent acetoxy groups, R2, R4, R6, R8 and R9 represent hydrogen atoms and X represents a fluorine atom) at a yield of 3% and β-tetraacetyl-2-deoxy-2-fluoromannose (β-TA-FDM of Fig. 15; in formula Chem. 5, R1, R5, R7 and R10 represent acetoxy groups, R2, R4, R6, R8 and R9 represent hydrogen atoms and X represents a fluorine atom) at a yield of 50%.

### Example 26

In the above implementation method, when a temperature of 300°C and a pressure of 5 MPa were set, fluorine-substituted compounds were obtained with a total yield of 20%, and pyran compounds with a total yield of 0% (Fig. 15). Here, the breakdown thereof was, as shown in Fig. 15, β-tetraacetyl-2-deoxy-2-fluoroglucose (β-TA-FDG of Fig. 15; in formula Chem. 4, R1, R5, R7 and R10 represent acetoxy groups, R2, R4, R6, R8 and R9 represent hydrogen atoms and X represents a fluorine atom) at a yield of 5%, tetraacetyl-4,5 dihydro-2H-pyran (2H-PR of Fig. 15; in formula Chem. 6, R1, R6, R7 and R10 represent acetoxy groups, R2, R4, R8 and R9 represent hydrogen atoms) at a yield of 0%, tetraacetyl-4,5 dihydro-4H-pyran (4H-PR of Fig. 15; in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 15; in formula Chem. 4, R1, R5, R7 and R10 represent acetoxy groups, R2, R4, R6, R8 and R9 represent hydrogen atoms and X represents a fluorine atom) at a yield of 1% and β-tetraacetyl-2-deoxy-2-fluoromannose (β-TA-FDM of Fig. 15; in formula Chem. 5, R1, R5, R7 and R10 represent acetoxy groups, R2, R4, R6, R8 and R9 represent hydrogen atoms and X represents a fluorine atom) at a yield of 13%.

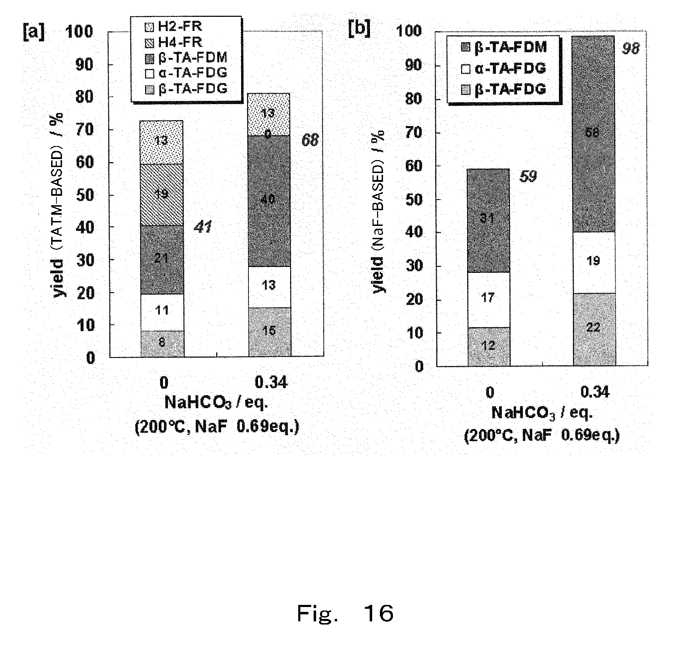

Furthermore, when the raw material β-TATM was dissolved in a solvent of ethanol/water = 50/50 (weight ratio) and 0.69 equivalents of sodium fluoride (in formula Chem. 3, M represents a sodium atom and X represents a fluorine atom), which reagent per se can be used as a PET reagent for bones, was used as the fluoride agent to perform the effects of an additive of sodium hydrogen carbonate (sodium bicarbonate) that is harmless to human and organisms, the results shown in Examples 27 and 28 described below and in Fig. 16 were obtained.

### Example 27

In the case of 0 equivalents sodium hydrogen carbonate in the above implementation method, when a temperature of 200°C and a pressure of 5 MPa were set, fluorine-substituted compounds were obtained with a total yield of 41%, and pyran compounds with a total yield of 32% (Fig. 16(a)). Here, the breakdown thereof was, as shown in Fig. 16(a), β-tetraacetyl-2-deoxy-2-fluoroglucose (β-TA-FDG of Fig. 16(a); in formula Chem. 4, R1, R5, R7 and R10 represent acetoxy groups, R2, R4, R6, R8 and R9 represent hydrogen atoms and X represents a fluorine atom) at a yield of 8%, tetraacetyl-4,5 dihydro-2H-pyran (2H-PR of Fig. 16(a); in formula Chem. 6, R1, R6, R7 and R10 represent acetoxy groups, R2, R4, R8 and R9 represent hydrogen atoms) at a yield of 13%, tetraacetyl-4,5 dihydro-4H-pyran (4H-PR of Fig. 16(a); in formula Chem. 7, R1, R6, R7 and R10 represent acetoxy groups, R2, R4, R8 and R9 represent hydrogen atoms) at a yield of 19%, α-tetraacetyl-2-deoxy-2-fluoroglucose (α-TA-FDG of Fig. 16(a); in formula Chem. 4, R1, R5, R7 and R10 represent acetoxy groups, R2, R4, R6, R8 and R9 represent hydrogen atoms and X represents a fluorine atom) at a yield of 11% and β-tetraacetyl-2-deoxy-2-fluoromannose (β-TA-FDM of Fig. 16(a); in formula Chem. 5, R1, R5, R7 and R10 represent acetoxy groups, R2, R4, R6, R8 and R9 represent hydrogen atoms and X represents a fluorine atom) at a yield of 21%.

Here, as these yields were TATM-based, when the yields were determined based on NaF, as in Fig. 16(b), fluorine-substituted compounds had a total yield of 59%, β-tetraacetyl-2-deoxy-2-fluoroglucose (β-TA-FDG of Fig. 16(b); in formula Chem. 4, R1, R5, R7 and R10 represent acetoxy groups, R2, R4, R6, R8 and R9 represent hydrogen atoms and X represents a fluorine atom) a yield of 12%, α-tetraacetyl-2-deoxy-2-fluoroglucose (α-TA-FDG of Fig. 16(b); in formula Chem. 4, R1, R5, R7 and R10 represent acetoxy groups, R2, R4, R6, R8 and R9 represent hydrogen atoms and X represents a fluorine atom) a yield of 17% and β-tetraacetyl-2-deoxy-2-fluoromannose (β-TA-FDM of Fig. 16(b); in formula Chem. 5, R1, R5, R7 and R10 represent acetoxy groups, R2, R4, R6, R8 and R9 represent hydrogen atoms and X represents a fluorine atom) a yield of 31%.

### Example 28

In the case of 0.34 equivalents sodium hydrogen carbonate in the above implementation method, when a temperature of 200°C and a pressure of 5 MPa were set, fluorine-substituted compounds were obtained with a total yield of 68%, and pyran compounds with a total yield of 13% (Fig. 16(a)). Here, the breakdown thereof was, as shown in Fig. 16(a), β-tetraacetyl-2-deoxy-2-fluoroglucose (β-TA-FDG of Fig. 16(a); in formula Chem. 4, R1, R5, R7 and R10 represent acetoxy groups, R2, R4, R6, R8 and R9 represent hydrogen atoms and X represents a fluorine atom) at a yield of 15%, tetraacetyl-4,5 dihydro-2H-pyran (2H-PR of Fig. 16(a); in formula Chem. 6, R1, R6, R7 and R10 represent acetoxy groups, R2, R4, R8 and R9 represent hydrogen atoms) at a yield of 13%, tetraacetyl-4,5 dihydro-4H-pyran (4H-PR of Fig. 16(a); in formula Chem. 7, R1, R6, R7 and R10 represent acetoxy groups, R2, R4, R8 and R9 represent hydrogen atoms) at a yield of 0%, α-tetraacetyl-2-deoxy-2-fluoroglucose (α-TA-FDG of Fig. 16(a); in formula Chem. 4, R1, R5, R7 and R10 represent acetoxy groups, R2, R4, R6, R8 and R9 represent hydrogen atoms and X represents a fluorine atom) at a yield of 13% and β-tetraacetyl-2-deoxy-2-fluoromannose (β-TA-FDM of Fig. 16(a); in formula Chem. 5, R1, R5, R7 and R10 represent acetoxy groups, R2, R4, R6, R8 and R9 represent hydrogen atoms and X represents a fluorine atom) at a yield of 40%.

Here, as these yields were TATM-based, when the yields were determined based on NaF, as in Fig. 16(b), fluorine-substituted compound had a total yield of 98%, β-tetraacetyl-2-deoxy-2-fluoroglucose (β-TA-FDG of Fig. 16(b); in formula Chem. 4, R1, R5, R7 and R10 represent acetoxy groups, R2, R4, R6, R8 and R9 represent hydrogen atoms and X represents a fluorine atom) a yield of 22%, α-tetraacetyl-2-deoxy-2-fluoroglucose (α-TA-FDG of Fig. 16(b); in formula Chem. 4, R1, R5, R7 and R10 represent acetoxy groups, R2, R4, R6, R8 and R9 represent hydrogen atoms and X represents a fluorine atom) a yield of 19% and β-tetraacetyl-2-deoxy-2-fluoromannose (β-TA-FDM of Fig. 16(b); in formula Chem. 5, R1, R5, R7 and R10 represent acetoxy groups, R2, R4, R6, R8 and R9 represent hydrogen atoms and X represents a fluorine atom) at a yield of 58%.

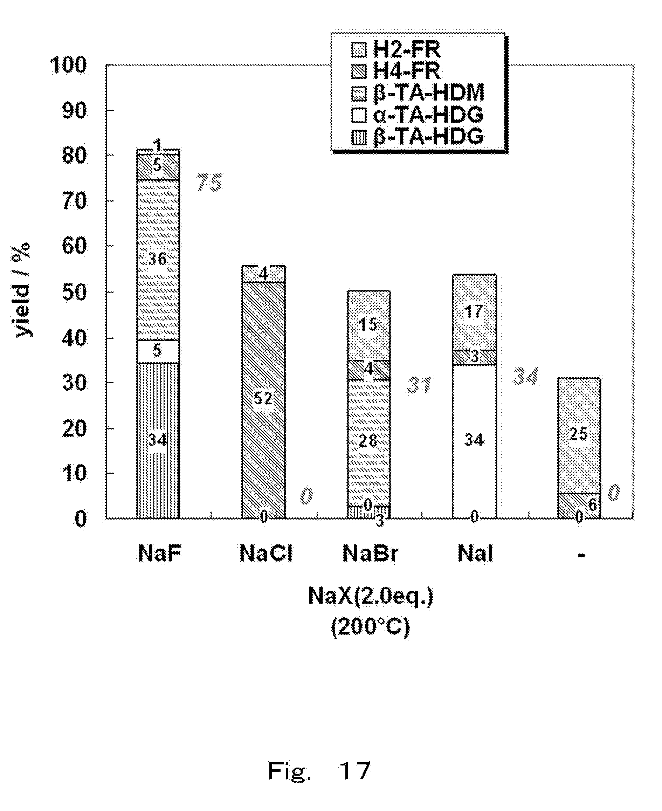

Furthermore, when the raw material β-TATM was dissolved in a solvent of ethanol/water = 50/50 (weight ratio) and 2 equivalents of sodium chloride (in formula Chem. 3, M represents a sodium atom and X represents a chlorine atom), sodium bromide (in formula Chem. 3, M represents a sodium atom and X represents a fluorine atom) and sodium iodide (in formula Chem. 3, M represents a sodium atom and X represents an iodine atom) were used as halide agents other than a fluoride agent to carry out halogenation, the results of Examples 29 to 31 and Fig. 17 were obtained. In addition, [the method] was carried out in a case with no halogen salt, and the results of Example 32 and Fig. 17 were obtained.

### Example 29

In the above implementation method, when the raw material β-TATM and sodium chloride (in formula Chem. 3, M represents a sodium atom and X represents a chlorine atom) were dissolved in a solvent of ethanol/water = 50/50 (weight ratio) and a temperature of 200°C and a pressure of 5 MPa were set, chlorine element-substituted compounds were obtained with a total yield of 0% and pyran compounds with a total yield of 56% (Fig. 17). Here, the breakdown thereof was, as shown in Fig. 17, β-tetraacetyl-2-deoxy-2-chloro glucose (β-TA-HDG of Fig. 17; in formula Chem. 4, R1, R5, R7 and R10 represent acetoxy groups, R2, R4, R6, R8 and R9 represent hydrogen atoms and X represents a chlorine atom) at a yield of 0%, tetraacetyl-4,5 dihydro-2H-pyran (2H-PR of Fig. 17; in formula Chem. 6, R1, R6, R7 and R10 represent acetoxy groups, R2, R4, R8 and R9 represent hydrogen atoms) at a yield of 4%, tetraacetyl-4,5 dihydro-4H-pyran (4H-PR of Fig. 17; in formula Chem. 7, R1, R6, R7 and R10 represent acetoxy groups, R2, R4, R8 and R9 represent hydrogen atoms) at a yield of 52%, α-tetraacetyl-2-deoxy-2-chloro glucose (α-TA-HDG of Fig. 17; in formula Chem. 4, R1, R5, R7 and R10 represent acetoxy groups, R2, R4, R6, R8 and R9 represent hydrogen atoms and X represents a chlorine atom) at a yield of 0% and β-tetraacetyl-2-deoxy-2-chloro mannose (β-TA-HDM of Fig. 17; in formula Chem. 5, R1, R5, R7 and R10 represent acetoxy groups, R2, R4, R6, R8 and R9 represent hydrogen atoms and X represents a chlorine atom) at a yield of 0%.

### Example 30

In the above implementation method, when the raw material β-TATM and sodium bromide (in formula Chem. 3, M represents a sodium atom and X represents a fluorine atom) were dissolved in a solvent of ethanol/water = 50/50 (weight ratio) and a temperature of 200°C and a pressure of 5 MPa were set, bromine-substituted compounds were obtained with a total yield of 31%, and pyran compounds with a total yield of 19% (Fig. 17). Here, the breakdown thereof was, as shown in Fig. 17, β-tetraacetyl-2-deoxy-2-bromo glucose (β-TA-HDG of Fig. 17; in formula Chem. 4, R1, R5, R7 and R10 represent acetoxy groups, R2, R4, R6, R8 and R9 represent hydrogen atoms and X represents a bromine atom) at a yield of 3%, tetraacetyl-4,5 dihydro-2H-pyran (2H-PR of Fig. 17; in formula Chem. 6, R1, R6, R7 and R10 represent acetoxy groups, R2, R4, R8 and R9 represent hydrogen atoms) at a yield of 15%, tetraacetyl-4,5 dihydro-4H-pyran (4H-PR of Fig. 17; in formula Chem. 7, R1, R6, R7 and R10 represent acetoxy groups, R2, R4, R8 and R9 represent hydrogen atoms) at a yield of 4%, α-tetraacetyl-2-deoxy-2-bromo glucose (α-TA-HDG of Fig. 17; in formula Chem. 4, R1, R5, R7 and R10 represent acetoxy groups, R2, R4, R6, R8 and R9 represent hydrogen atoms and X represents a bromine atom) at a yield of 0% and β-tetraacetyl-2-deoxy-2-bromo mannose (β-TA-HDM of Fig. 17; in formula Chem. 5, R1, R5, R7 and R10 represent acetoxy groups, R2, R4, R6, R8 and R9 represent hydrogen atoms and X represents a bromine atom) at a yield of 28%.

### Example 31

In the above implementation method, when the raw material β-TATM and sodium iodide (in formula Chem. 3, M represents a sodium atom and X represents an iodine atom) were dissolved in a solvent of ethanol/water = 50/50 (weight ratio) and a temperature of 200°C and a pressure of 5 MPa were set, iodine-substituted compounds were obtained with a total yield of 34%, and pyran compounds with a total yield of 20% (Fig. 17). Here, the breakdown thereof was, as shown in Fig. 17, β-tetraacetyl-2-deoxy-2-iodo glucose (β-TA-HDG of Fig. 17; in formula Chem. 4, R1, R5, R7 and R10 represent acetoxy groups, R2, R4, R6, R8 and R9 represent hydrogen atoms and X represents an iodine atom) at a yield of 0%, tetraacetyl-4,5 dihydro-2H-pyran (2H-PR of Fig. 17; in formula Chem. 6, R1, R6, R7 and R10 represent acetoxy groups, R2, R4, R8 and R9 represent hydrogen atoms) at a yield of 17%, tetraacetyl-4,5 dihydro-4H-pyran (4H-PR of Fig. 17; in formula Chem. 7, R1, R6, R7 and R10 represent acetoxy groups, R2, R4, R8 and R9 represent hydrogen atoms) at a yield of 3%, α-tetraacetyl-2-deoxy-2-iodo glucose (α-TA-HDG of Fig. 17; in formula Chem. 4, R1, R5, R7 and R10 represent acetoxy groups, R2, R4, R6, R8 and R9 represent hydrogen atoms and X represents an iodine atom) at a yield of 0% and β-tetraacetyl-2-deoxy-2-iodo mannose (β-TA-HDM of Fig. 17; in formula Chem. 5, R1, R5, R7 and R10 represent acetoxy groups, R2, R4, R6, R8 and R9 represent hydrogen atoms and X represents an iodine atom) at a yield of 34%.

### Example 32

In the above implementation method, when the raw material β-TATM alone with no addition of halide salt was dissolved in a solvent of ethanol/water = 50/50 (weight ratio) and a temperature of 200°C and a pressure of 5 MPa were set, pyran compounds were obtained with a total yield of 31% (Fig. 17). Here, the breakdown thereof was, as shown in Fig. 17, tetraacetyl-4,5 dihydro-2H-pyran (2H-PR of Fig. 17; in formula Chem. 6, R1, R6, R7 and R10 represent acetoxy groups, R2, R4, R8 and R9 represent hydrogen atoms) at a yield of 25% and tetraacetyl-4,5 dihydro-4H-pyran (4H-PR of Fig. 17; in formula Chem. 7, R1, R6, R7 and R10 represent acetoxy groups, R2, R4, R8 and R9 represent hydrogen atoms) at a yield of 6%.

Meanwhile, with 1,2:5,6-di-O-isopropylidene-α-D-allofuranose-3-triflate (α-DIAT; in formula Chem. 8, R2, R3, R10 and R11 represent isopropylidenedioxy groups, R1, R4, R5 and R9 represent hydrogen atoms and L represents OTf group) as raw material, fluorination into 1,2:5,6-di-O-isopropylidene-α-D-3-fluoro-3-deoxy-glucofuranose (α-DIFG; in formula Chem. 9, R2, R3, R10 and R11 represent isopropylidenedioxy groups, R1, R4, R5 and R9 represent hydrogen atoms and L represents F group) and 1,2:5,6-di-O-isopropylidene-α-D-3-deoxy-3-fluoro-allofuranose (α-DIFA; in formula Chem. 10, R2, R3, R10 and R11 represent isopropylidenedioxy groups, R1, R4, R5 and R9 represent hydrogen atoms and L represents F group) was carried out. [the reaction was] Using the circulating-type high temperature and high pressure reaction device of Fig. 9 with the synthesis conditions: no catalyst, a temperature of 100 to 200°C, a pressure of 5 MPa and a retention time of 68 seconds.

To an device comprising the circulating-type high-temperature and high-pressure liquid in-situ infrared spectrophotometer of Fig. 7, first, the main body (major parts) of the circulating-type high temperature and high pressure reaction device of Fig. 9 was fitted at a prescribed temperature and a prescribed pressure, and a pure water solution was sent to a windowless cell (Tee 1) by a pump 5 at a flow rate of 5.0 ml/min. Thereafter, in an aqueous solution of ethanol prepared beforehand at a proportion of 25g water to 25 g ethanol, 1 g (0.393 mmol) of a dichloromethane solution of the substrate α-DIAT (0.39 mol/kg) and 0.033g (0.08 mmol) of sodium fluoride (in formula Chem. 3, M represents a sodium atom and X represents a fluorine atom) were dissolved, toluene was added as an internal standard (5 mol% of the substrate), and 0.693 ml/min of the mixed solution was sent to the windowless cell (Tee 1) with the pump 6 (aqueous solution concentration after mixing: 0.06 mmol/kg). After substrate solution sending, 1 ml of discharge aqueous solution was collected from the back-pressure valve 20 minutes later.

To 1 ml of the aqueous solution recovered, 1 ml of acetone was added, [the solution was] shaken, compositional analysis was carried out with a GC/MS analyzer (HP6890, manufactured by Hewlett Packard; column: HP-5; injection port temperature: 250°C; initial column temperature: 75°C (retention time: 0.5 minutes); temperature rise speed: 60°C/minute; final column temperature: 250°C (retention time: 2 minutes)), and the obtained mass spectrum was identified with a matching degree of 90% or greater with the Willey database. In addition, quantitative determination and, if a commercial reagent exists, qualitative determination were carried out by GC-FID (GC6890, manufactured by Agilent; column: HP-5; injection port temperature: 250°C; split ratio: 5.61; initial column temperature: 75°C (retention time: 0.5 minutes); temperature rise speed: 60°C/minute; final column temperature: 250°C (retention time: 2 minutes)) with toluene as an internal standard.

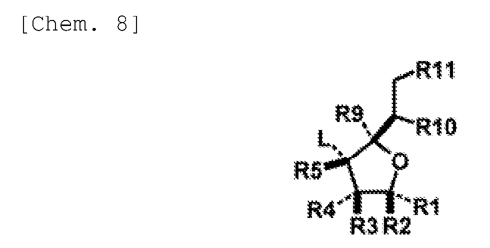

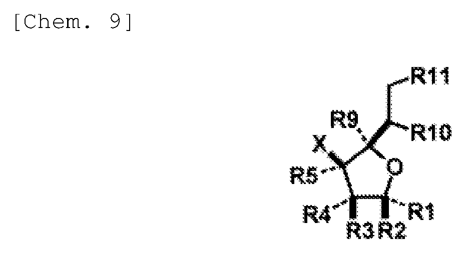

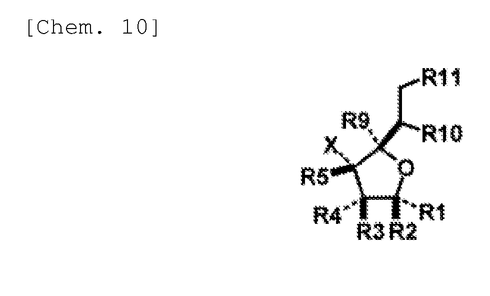

### Example 33

In the above implementation method, when a temperature of 100°C and a pressure of 5 MPa were set, fluorine-substituted compounds were obtained with a total yield of 8%, and furan compounds with a total yield of 0%. Here, the breakdown thereof was 1,2:5,6-di-O-isopropylidene-α-D-3-fluoro-3-deoxy-glucofuranose (α-DIFG; in formula Chem. 9, R2, R3, R10 and R11 represent isopropylidenedioxy groups, R1, R4, R5 and R9 represent hydrogen atoms and L represents F group) at a yield of 0% and 1,2:5,6-di-O-isopropylidene-α-D-3-deoxy-3-fluoro-allofuranose (α-DIFA; in formula Chem. 9, R2, R3, R10 and R11 represent isopropylidenedioxy groups, R1, R4, R5 and R9 represent hydrogen atoms and L represents F group) at a yield of 0%, which were not obtained, α-D-3-fluoro-3-deoxy-glucofuranose (in formula Chem. 9, R2, R3, R10 and R11 represent hydroxyl groups, R1, R4, R5 and R9 represent hydrogen atoms and L represents F group) at a yield of 8% and α-D-3-deoxy-3-fluoro-allofuranose (in formula Chem. 10, R2, R3, R10 and R11 represent hydroxyl groups, R1, R4, R5 and R9 represent hydrogen atoms and L represents F group) at a yield of 0%, which had the protecting groups isopropylidenedioxy group eliminated.

### Example 34

In the above implementation method, when a temperature of 200°C and a pressure of 5 MPa were set, fluorine-substituted compounds were obtained with a total yield of 18%, and furan compounds with a total yield of 0%. Here, the breakdown thereof was 1,2:5,6-di-O-isopropylidene-α-D-3-fluoro-3-deoxy-glucofuranose (α-DIFG, in formula Chem. 9, R2, R3, R10 and R11 represent isopropylidenedioxy groups, R1, R4, R5 and R9 represent hydrogen atoms and L represents F group) at a yield of 0% and 1,2:5,6-di-O- isopropylidene-α-D-3-deoxy-3-fluoro-allofuranose (α-DIFA, in formula Chem. 9, R2, R3, R10 and R11 represent isopropylidenedioxy groups, R1, R4, R5 and R9 represent hydrogen atoms and L represents F group) at a yield of 0%, which were not obtained, α-D-3-fluoro-3-deoxy-glucofuranose (in formula Chem. 9, R2, R3, R10 and R11 represent hydroxyl groups, R1, R4, R5 and R9 represent hydrogen atoms and L represents F group) at a yield of 18% and α-D-3-deoxy-3-fluoro-allofuranose (in formula Chem. 10, R2, R3, R10 and R11 represent hydroxyl groups, R1, R4, R5 and R9 represent hydrogen atoms and L represents F group) at a yield of 0%, which had the protecting groups isopropylidenedioxy group eliminated.

From the above examples, it became clear that, with a high temperature and high pressure water as the reaction solvent and no catalyst, a halogen-substituted saccharide could be synthesized at high yield, while reducing the amount of waste such as aprotic organic solvent, phase transfer catalyst and solvent due to solvent substitution. In addition, it became clear that the present invention is also useful as a simple, continuous separation method in which, after halogen-substituted saccharide synthesis, water is poured over the recovered aqueous solution for decantation which is separated into solid-liquid bilayer solutions, then, the solid phase containing the halogen-substituted saccharide is separated from liquid and recovered, whereas water is separated and recovered from the aqueous layer.

### INDUSTRIAL APPLICABILITY

As described above, the present invention relates to a method for synthesizing a halogen-substituted saccharide from a leaving group-substituted saccharide and a halide salt without using an aprotic organic solvent or a phase transfer catalyst, without solvent substitution, with a high temperature and high pressure liquid as the reaction solvent and with no catalyst; and a reaction composition of same; while, by prior art methods, the synthesis of a halogen-substituted saccharide from a leaving group-substituted saccharide and a halide salt require the use of a phase transfer catalyst in addition to an aprotic organic solvent, the reaction required solvent substitution to be carried out, and an environmentally friendly type of process that is friendly to humans, organisms and the environment, in which aprotic organic solvent/catalyst have been removed, could not be realized, by using the subcritical fluid/supercritical fluid shown in the present invention, synthesizing a halogen-substituted saccharide has become possible, with no addition of a catalyst, without using an aprotic organic solvent, while reducing the amount of energy consumption and the amount of waste, at high-speed, continuously and selectively. This exerts the merit that halogen-substituted saccharide useful as a medicinal product beneficial to humans can be produced in a short time, in large amounts and continuously. In addition, after halogen-substituted saccharide synthesis, pouring water over the recovered aqueous solution for decantation which is separated into solid-liquid bilayer solutions, then, liquid-separating and recovering the solid layer containing the halogen-substituted saccharide, while recovering water from the aqueous layer, allows water to be recycled. From these, by simplifying the synthesis/separation process, the initial cost and running cost of the process can be compressed. Furthermore, the post-processing of neutralization treatment is also unnecessary such that an environmentally harmonious production becomes possible. The present invention is one that may substitute existing production processes as a novel mass production process for halogen-substituted saccharide useful as a medicinal product.

### EXPLANATION OF REFERENCE NUMERALS

- 1: Tee (one-open side 4 mm diameter screw threading)
- 2: 4 mm diameter × 5.0 mm L hexagonal screw
- 3: One-ring ferrule-fitted male screw
- 4: SUS316 tube
- 5: Water solution-sending pump
- 6: Reactant solution-sending pump
- 7: Wash water solution-sending pump
- 8: Cooling flange (cooling water circulates)
- 9: Water-heating coil
- 10: Reactant inlet tube
- 11: Temperature sensor
- 12: Temperature sensor sheath
- 13: Furnace body
- 14: High temperature and high pressure flow-cell
- 15: ZnSe window
- 16: Solvent inlet tube
- 17: Discharge piping
- 18: Tee
- 19: Back-pressure valve
- 21: Aqueous solution
- 22: Wash water
- 23: Aqueous solution pump
- 24: Washing pure water solution-sending pump
- 25: Furnace body heating system
- 26: Furnace body
- 27: High temperature and high pressure infrared flow-cell
- 28: Cooling water (inlet)
- 29: Cooling water (outlet)
- 30: Back-pressure valve
- 31: Discharge aqueous solution receptacle
- 32: Moving mirror
- 33: Moving mirror
- 34: Interferometer
- 35: Light source
- 36: Infrared laser
- 37: MCT photodetector
- 38: TGS photodetector
- 39: Analysis monitor
- 40: Reactant solution-sending pump
- 41: Substrate solution-sending pump
- 42: Water solution-sending pump
- 43: Reaction tee
- 44: Piping
- 45: Mixing tee
- 46: Discharge piping
- 47: Cooler
- 48: Back-pressure valve
- 49: Recovery container
- 50: Temperature sensor
- 51: Temperature sensor

## Claims

1. An aqueous solution of halogen-substituted saccharide which is synthesized by a halogen-substituted saccharide synthesis reaction from a leaving group-substituted protected saccharide or a leaving group-substituted saccharide and a halide salt, and contains a halogen substitution reaction product or contains a halogen-substituted saccharide for a tracer in positron emission tomography (PET), and in which no catalyst and no aprotic organic solvent harmful to a human and an organism remain.

2. A method for producing a halogen-substituted saccharide, using a subcritical fluid or a supercritical fluid in a high-temperature and high-pressure state as the reaction solvent to carry out, from a leaving group-substituted protected saccharide and a halide salt, halogen substitution and deprotection in a single step without substituting the solvent and with no catalyst, thereby synthesizing a halogen-substituted saccharide selectively.

3. A method for producing a halogen-substituted saccharide, using a subcritical fluid or a supercritical fluid in a high-temperature and high-pressure state as the reaction solvent to carry out, from a leaving group-substituted saccharide and a halide salt, halogen substitution and deprotection in a single step without substituting the solvent and with no catalyst, thereby synthesizing a halogen-substituted saccharide selectively.

4. The method according to Claim 2 or 3, wherein a subcritical fluid or a supercritical fluid at a temperature of 100 to 400°C and a pressure of 0.1 to 40 Mpa is used as the reaction solvent.

5. The method according to Claim 2 or 3, wherein water, ethanol, another inorganic solvent, or an organic solvent or a mixed solvent of an inorganic solvent and an organic solvent, is used as the subcritical fluid or the supercritical fluid.

6. The method according to Claim 2 or 3, wherein the synthesis reaction is carried out by introducing the substrate and the reaction solvent into a circulating-type high-temperature and high-pressure device, and varying the reaction time in a range of 3 to 180 seconds.

7. The method according to Claim 2 or 3, wherein an additive of sodium hydrogen carbonate (sodium bicarbonate), which is harmless to a human and an organism, is added to accelerate the synthesis reaction.

8. A halogen-substituted saccharide synthesizer, which is a micro reaction system in which a flow-path space therein is a micro space, the synthesizer being provided with a water solution-sending pump for sending a water solution, a water heating coil, a high-temperature and high-pressure flow-cell, a reactant solution-sending pump for sending a substrate solution, a furnace body, a reactant inlet tube for introducing a reactant into the furnace body, a discharge solution line for discharging the reaction solution, a cooling flange and a back-pressure valve for setting the pressure, wherein
the flow of an aqueous solution in which a leaving group-substituted protected saccharide and a halogen salt have been dissolved or the flow of an aqueous solution in which a leaving group-substituted saccharide and a halogen salt have been dissolved is collided at a right angle against the flow of high temperature and high pressure water in the high-temperature and high-pressure flow-cell, thereby synthesizing halogen-substituted saccharide selectively.

9. A method of a simple continuous solid-liquid separation of a solid containing a halogen-substituted saccharide, by which, after halogen-substituted saccharide synthesis, water is poured onto the recovered aqueous solution for decantation, and after separation into solid-liquid bilayer solutions, the solid containing the halogen-substituted saccharide is separated and recovered in one operation.
